# EUROPÄISCHE PATENTSCHRIFT

(11) **EP 2 214 760 B1**
(45) Veröffentlichungstag und Bekanntmachung des Hinweises auf die Patenterteilung: **14.08.2013**
(21) Anmeldenummer: 08852601.7
(22) Anmeldetag: 29.10.2008
(51) Int. Cl.: A61M 15/00

(54) **DOSIERVORRICHTUNG ZUR INHALIERUNG EINER PULVERFÖRMIGEN SUBSTANZ**
METERING DEVICE FOR INHALING A POWDERY SUBSTANCE
DISPOSITIF DE DOSAGE DESTINÉ À L'INHALATION D'UNE SUBSTANCE PULVÉRULENTE

(30) Priorität: 22.11.2007 DE 102007056263
(43) Veröffentlichungstag der Anmeldung: 11.08.2010
(73) Patentinhaber: Sanofi SA, 1214 Vernier (CH)
(72) Erfinder: VON SCHUCKMANN, Alfred, 47627 Kevelaer (DE); KAMLAG, Yorick, 82396 Pähl (DE); MAYER, Stefan, 79098 Freiburg i. Br. (DE); SANDELL, Dennis, 27562 Blentarp (SE)
(74) Vertreter: Epping - Hermann - Fischer
(86) Internationale Anmeldenummer: PCT/EP2008/064652
(87) Internationale Veröffentlichungsnummer: WO 2009/065707

(56) Entgegenhaltungen:
- EP-A- 0 505 321
- WO-A-02/26299
- WO-A-2006/021546
- DE-A1-102006 029 753

## Beschreibung

Die Erfindung betrifft eine vom Saugluftstrom des Benutzers aktivierbare Dosiervorrichtung zur Inhalierung einer pulverförmigen Substanz, insbesondere medizinischer Art, gemäß Gattungsbegriff des Hauptanspruches.

Eine Dosiervorrichtung der in Rede stehenden Art ist aus der WO 2006/021546 A1 bekannt. Die in der Dosierkammer abgeteilte Substanzmenge wird in eine verschlossene Entleerungsbereitschaftsstellung bewegt. Infolge eines Einatmens bewegt sich ein Kolben und öffnet die Dosierkammer. Diese steht hiernach im Anschluss an einen Luftströmungsweg zum Räumen der abgeteilten Substanzmenge aus der Dosierkammer und zur Überführung in den einzusaugenden Luftstrom.

Im Hinblick auf den bekannten Stand der Technik wird eine technische Problematik der Erfindung darin gesehen, eine Dosiervorrichtung der in Rede stehenden Art im Hinblick auf den Inhaliervorgang, insbesondere auf die Bewegung des Kolbens in die Entleerungs-Freigabestellung in vorteilhafter Weise weiterzubilden.

Diese Problematik ist im Wesentlichen durch den Gegenstand des Anspruches 1 gelöst, wobei darauf abgestellt ist, dass der im oberen Bereich tellerförmig gestaltete Kolben mit von der Tellerunterseite ausgehenden, die Dosierkammer in die Entleerungsbereitschaftsstellung verschließenden Zungen ausgestattet ist, welche bei der vom Benutzersaugluftstrom veranlassten Kolbenverlagerung die Dosierkammer freigeben. Es ist zufolge dieser Ausgestaltung eine platzsparende und hinsichtlich der Kolbenausgestaltung gewichtssparende und leichtgängige Lösung gegeben. Der Kolben ist hinsichtlich seiner Funktionsabschnitte minimiert, weist entsprechend eine zur Verlagerung desselben dienende Kolbenfläche und die Dosierkammer in der Entleerungsbereitschaftsstellung überdeckende Zungen auf. Der Kolbenteller ist annähernd bevorzugt als Flächenbauteil gebildet, in weiterer Ausgestaltung kalottenartig mit einer der Kolbenbeaufschlagungsfläche abgewandten Kalottenöffnung. Der Kolbenteller weist hierbei eine Material- bzw. Wandungsstärke auf, die ausreichend ist zur Sicherstellung einer ausreichenden Eigenstabilität des Tellers, so bspw. 0,5 - 2 mm, weiter bspw. 1 mm. Einstückig und materialeinheitlich mit diesem Kolbenteller sind die Zungen ausgeformt. Diese erstrecken sich beidseitig einer zentralen Kolbenachse verlaufend ausgehend von der Kolbenbeaufschlagungsfläche. Die Zungen können hierbei weiter in einem Querschnitt rechteckig gestaltet sein, mit einer Querschnittslänge, die einem Mehrfachen der Querschnittsbreite entspricht. Alternativ können die Zungen im Querschnitt auch kreissegmentartig gestaltet sein, mit einer der Dosierkammer zugeordneten Flachseite. Durch die gewählte Ausgestaltung des Kolbens ist zur Verlagerung desselben relativ wenig Masse bei großer Angriffsfläche zu verlagern, was die Bewegung des Kolbens aus der Entleerungs-Bereitschaftsstellung in die Entleerungs-Freigabestellung mittels des Benutzer-Saugluftstromes erleichtert. Es bedarf entsprechend nur einer relativ geringen Saugluftstromenergie zur Freigabe der Dosierkammer. Darüber hinaus ist durch die schlanke Bauform des Kolbens eine erhöhte Luftenergie im Zuge der Inhalation erreichbar. Man kann zufolge der nur schmalen Zungen ohne erhöhte Reibung auch mehrere Dosierkammern überdecken. Der Dosierstab ist auch jederzeit leicht auszutauschen, z.B. zwecks Anpassung an eine Substanzendosis.

In vorteilhafter Weiterbildung ist vorgesehen, dass der obere Rand des Kolbens in seiner oberen Endstellung vor eine Ringwand tritt, die zu einer Ringkammer gehört, vorzugsweise hat deren Decke randseitig ausladende, vorstehende Flügel, welche zwischen sich Zwischenräume belassen. Dieser nachgeordnet ist ein Deckenabschnitt, der eine schrägstehende, bündelnde Prellwand darstellt. Der im Zuge der Inhalation, d.h. der Saugluftbeaufschlagung durch den Benutzer luftumströmte Kolben gibt weiter bevorzugt in der oberen, d.h. in der Entleerungs-Freigabestellung der Dosierkammer den Weg zu einer Ringkammer frei. Das zu inhalierende Pulver besteht bspw. aus einem saugstromtransportfähigen Grundkörper wie Laktose, das als Träger für oberflächenseitig anhaftende, mikronisierte Arzneimittel-Feinpartikel geeignet ist. Die pulverbehaftete Saugluft wird durch zwischen den deckelseitig nach radial außen ausladenden Flügeln ausgebildeten Zwischenräume abgesaugt, von wo aus sie leicht gebündelt in das Mundstück der Dosiervorrichtung tritt. Eine weitere, platzsparende Lösung ist durch teilweises Einsenken des Rastkopfes des Dosierstabes in die obere Vertiefung des Tellerkolbens erreicht, unter Einsenken in die erwähnte kalottenartige Höhlung des Kolbens. Der Dosierstab ist in einem mit der Verschlusskappe drehbaren Innenzylinder in Axialerstreckung des Innenzylinders verschiebbar gehaltert. Die Drehung des Innenzylinders wird auf den Dosierstab übertragen. Mantelwandseitig ist dieser Innenzylinder mit einem axial verlaufenden Kanal versehen, der von der Entleerungsseite der Dosierkammer ausgeht und in der Ringkammer endet, wobei zur Umlenkung der axialen Luftstrom-Richtung in die Umlaufebene der Umlenk-Abprallwandflügel vorgesehen sein kann. Letzterer ist entsprechend in axialer Verlängerung des Kanals deckelartig unter Belassung eines Radialaustritts angeordnet. Über diesen Kanal wird nach saugluftbedingtem Anheben des Kolbens und damit einhergehender Freigabe der Dosierkammer die abgeteilte Substanzdosis ausgesaugt und über eine Ringkammer dem, den Saugluftstrom aufbauenden Benutzer zugeführt. Bei einer üblichen Aussaugung der Dosierkammer in Radialrichtung erfolgt zunächst eine Umlenkung in den axial verlaufenden Kanal, dies entsprechend verbunden mit einer Prallplattenwirkung zur Auslösung von Grobpulverteilchen. In bevorzugter Ausgestaltung ist die Umlenkung aus der Radialströmung in die Axialströmung durch zwei unmittelbar hintereinander geschaltete Kanal-Umlenkbereiche erreicht, die jeweils eine Strömungsumlenkung von 45 Grad bewirken. So ist weiter bevorzugt ein unter einem Winkel von etwa 45 Grad zu einer quer zur Vorrichtungsachse ausgerichteten Ebene verlaufender Kanalzwischenabschnitt vorgesehen, der die Entleerungsseite der Dosierkammer mit dem axial verlaufenden Kanal verbindet.

Die Zungen sind an ihrem unteren freien Rand lippenartig gespalten zur Erzielung einer Klemmwirkung in Zusammenwirkung mit dem Dosierstab, bzw. zur Dichtzusammenwirkung mit einem Dichtsitz. Weiter besitzen die Zungen materialverstärkte Abdichtungsflächen, zum beidseitigen Verschluss der Dosierkammer in der Entleerungs-Bereitschaftsstellung. Nach Verlagerung des Dosierstabes mit der Dosierkammer in die Entleerungs-Bereitschaftsstellung kann ohne negative Folgen auch ohne durchgeführte Inhalation die Verschlusskappe wieder aufgeschraubt werden, was die Rückverlagerung des Dosierstabes zur Folge hat. Dadurch bedingt, dass lediglich die mit der Dosierkammer zusammenwirkenden Abdichtungsflächen materialverstärkt sind, sind die weiteren Axialabschnitte der Zungen in einem Querschnitt betrachtet um das Materialverstärkungsmaß beabstandet zur zugeordneten Breitfläche des Dosierstabes. Zufolge dieser Ausgestaltung sind im Zuge der saugluftbedingten Axialverlagerung die Reibkräfte des Kolbens mit den Zungen minimiert. Ein Einsaug-Strömungskanal, der auf die eine der beiden Zungen gerichtet ist, stellt eine optische Kontrolle dar, ob sich der Stab in der Entnahme-Bereitschaftsstellung befindet.

Die zu inhalierende Substanz ist in einer Vorratskammer bevorratet, in welche die Dosierkammer zur Befüllung derselben eintaucht. Um hier den Befüllvorgang der Dosierkammer zu unterstützen, weiter eine stets aufgelockerte, von der Dosierkammer durchsetzte oberste Schicht des Substanzvorrates zu erreichen, ist ein rotorartiger Flügel am unteren Rand des Innenzylinders gehaltert, so bspw. an diesen geclipst, welcher Flügel mit einer einwärts gerichteten statorförmigen Schulter der Vorratskammerwand zusammenwirkt. Hierüber ist das Nachbringen und auch die Dichte der Substanz in der Vorratskammer gleich haltbar. Hinzu kommt ein im Umfeld der Dosierkammer gegebener Auflockerungseffekt, der das Stocken von Substanzpartien ausschließt. Darüber hinaus ist der Rotor in Zusammenwirkung mit dem Stator so gestaltet, dass bei einer Rückbewegung der rotorartigen Flügel im Zuge des Wiederaufsetzens und Aufschraubens der Verschlusskappe bei einhergehendem Absenken der Dosierkammer in die Vorratskammer ein leichtes Andrücken der obersten Substanzschicht erreicht wird, um so einen vergleichmäßigten, der Dosierkammer zugeordneten obersten Substanzmengenbereich in der Vorratskammer anzubieten. Es ergibt sich eine Füllung der Dosierkammer, die sicher mit den Zungen eingeschlossen werden kann.

Schließlich erweist es sich noch von Vorteil, wenn im Bereich der Vorratskammerwand eine Füllstandsanzeige vorgesehen ist, welche die Füllung erkennen lässt. Diese kann in einfachster Ausgestaltung direkt gekoppelt sein an die Axialbewegung eines in der Vorratskammer angeordneten, die bevorratete Substanzmenge von unten in Richtung gegen den Innenzylinder belastenden Druckkolbens. Dieser wandert mit Substanzentnahme nach, was über die Füllstandsanzeige beobachtet werden kann.

Nachstehend ist die Erfindung anhand der beigefügten Zeichnung, welche lediglich ein Ausführungsbeispiel darstellt, näher erläutert. Es zeigt:
- Fig. 1: den Vertikalschnitt durch eine erfindungsgemäße Dosiervorrichtung in kappenverschlossener Grundstellung;
- Fig. 2: einen weiteren Vertikalschnitt gemäß der Linie II - II in Fig. 1;

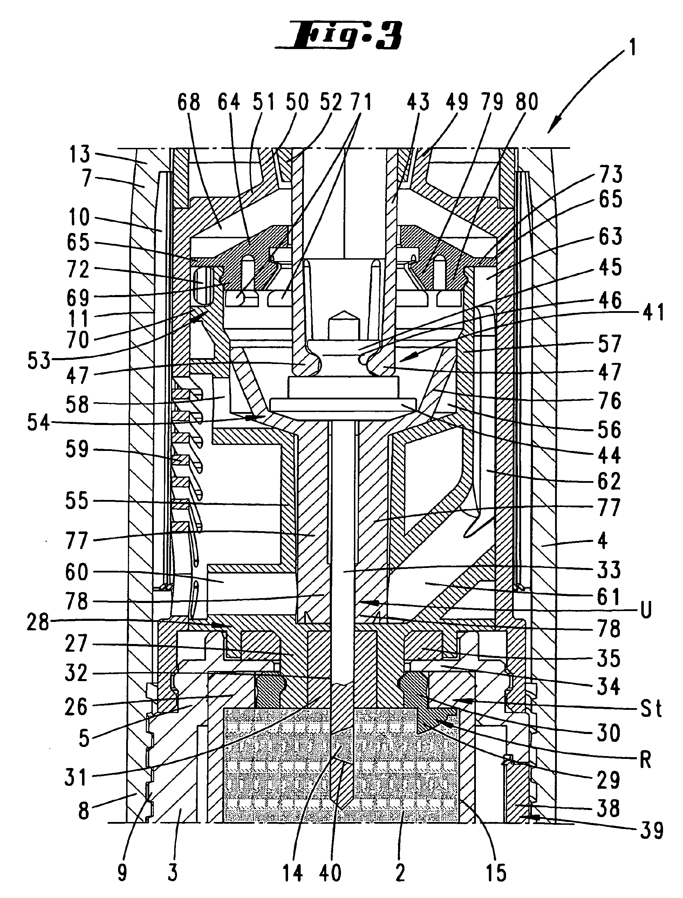

- Fig. 3: eine Herausvergrößerung eines oberen Bereichs der Vorrichtung gemäß Fig. 1;
- Fig. 4: eine der Fig.1 entsprechende Schnittdarstellung, die Situation bei nahezu entleerter Vorratskammer für die zu inhalierende Substanz betreffend;

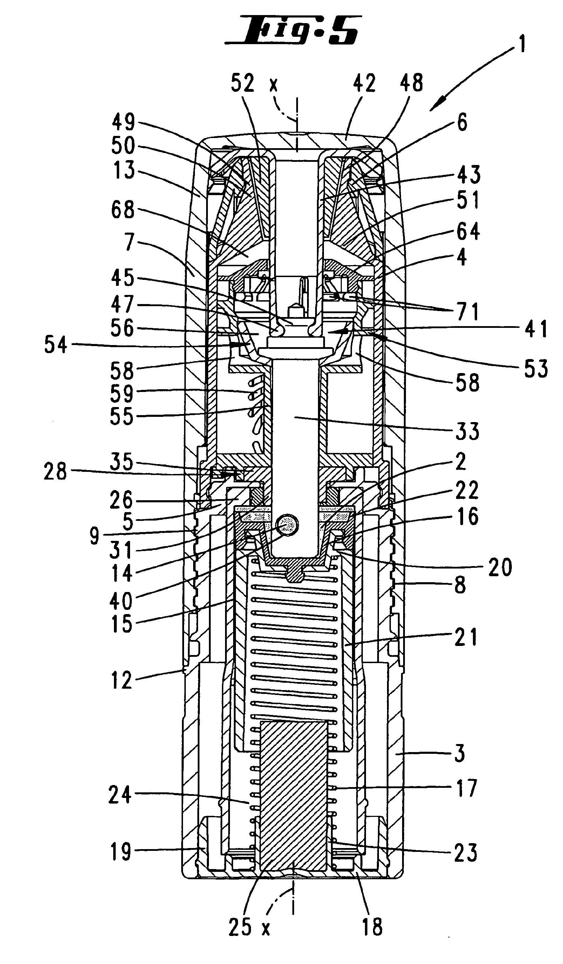

- Fig. 5: den Schnitt gemäß der Linie V - V in Fig. 4;
- Fig. 6: eine weitere der Fig.1 entsprechende Darstellung, im Zuge der Abnahme der Verschlusskappe;
- Fig. 7: den Schnitt gemäß der Linie VII - VII in Fig. 6;

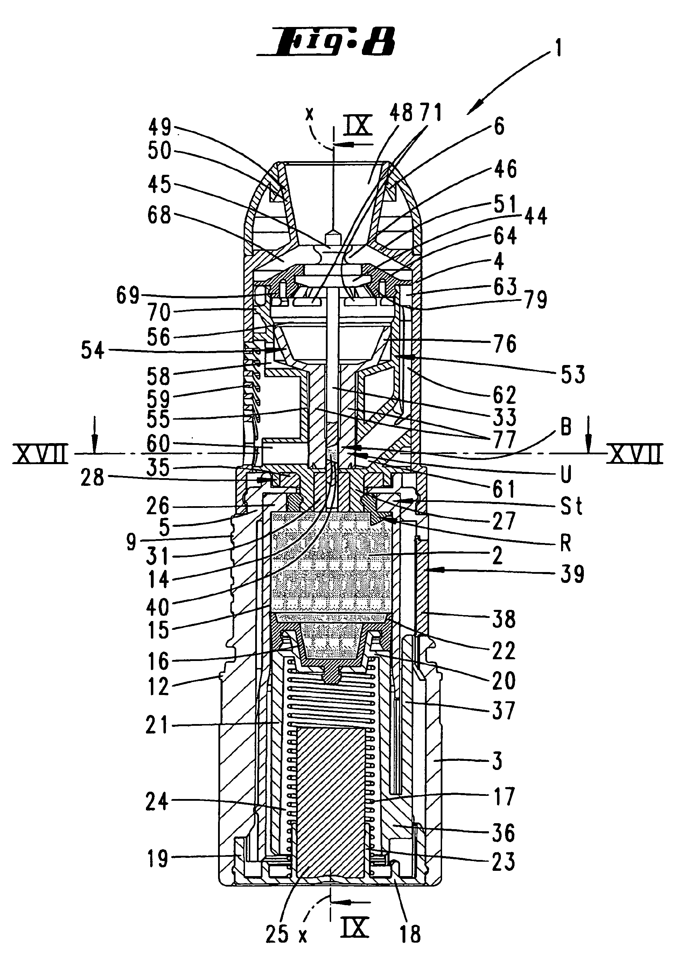

- Fig. 8: den Vertikalschnitt gemäß Fig. 1, jedoch nach Abnahme der Verschlusskappe und hierdurch bedingter Verlagerung einer Dosierkammer in die Entleerungs-Bereitschaftsstellung;

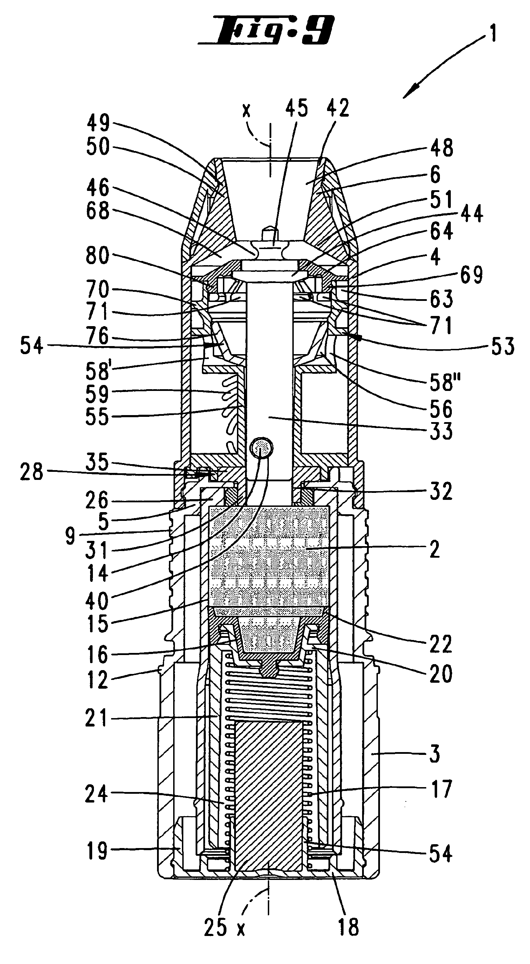

- Fig. 9: den Schnitt gemäß der Linie IX - IX in Fig. 8;

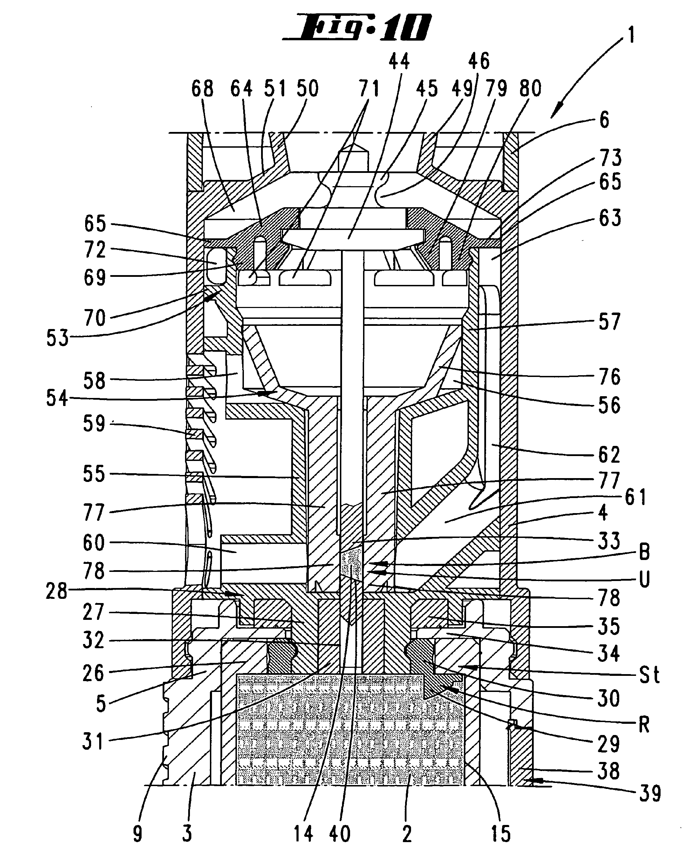

- Fig. 10: eine der Fig. 3 entsprechende Ausschnittdarstellung, die Situation gemäß Fig. 8 betreffend;

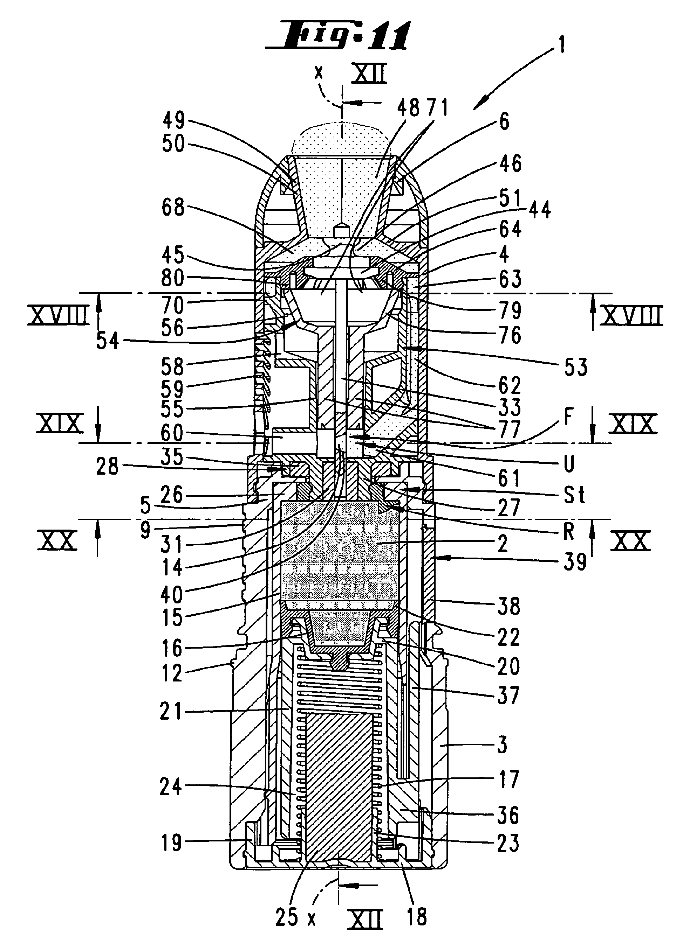

- Fig. 11: eine Folgedarstellung der Fig. 8, jedoch eine Stellung im Zuge der Inhalation betreffend;

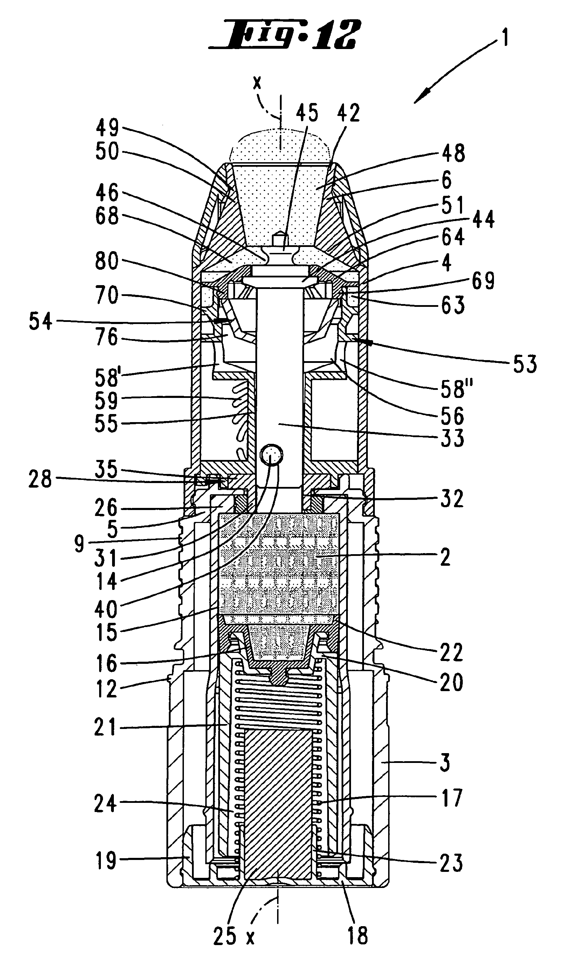

- Fig. 12: den Schnitt gemäß der Linie XII - XII in Fig. 11;

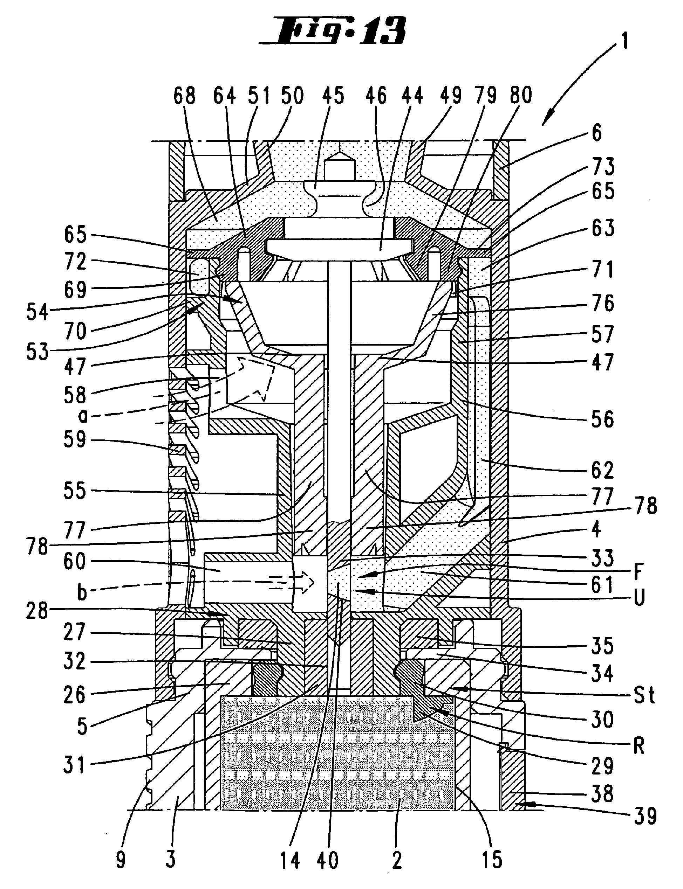

- Fig. 13: eine weitere der Fig. 3 entsprechende Ausschnittdarstellung, jedoch die Situation gemäß Fig. 11 betreffend;

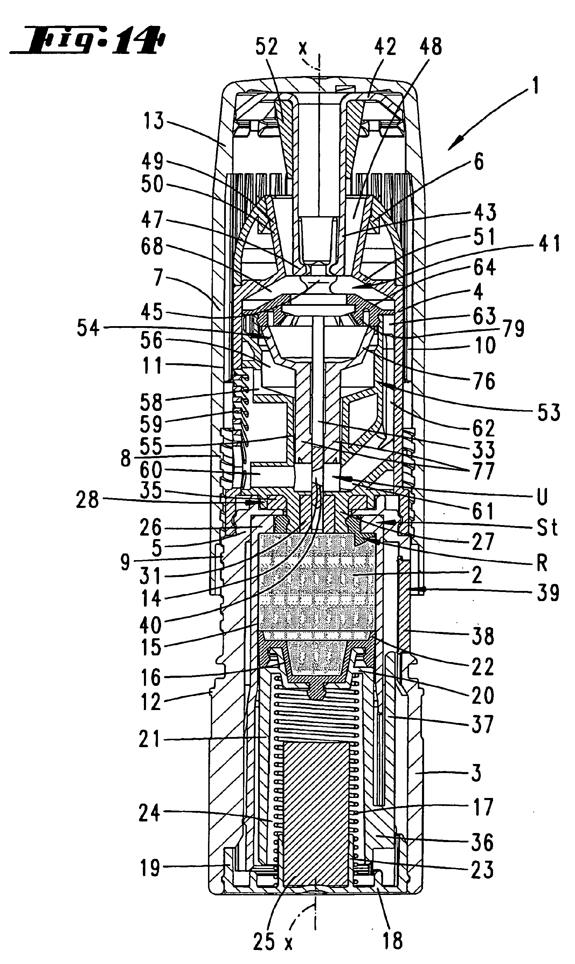

- Fig. 14: eine weitere der Fig.1 entsprechende Vertikalschnittdarstellung, eine Zwischenstellung im Zuge des Wiederaufsetzens der Verschlusskappe nach erfolgter Inhalation betreffend;

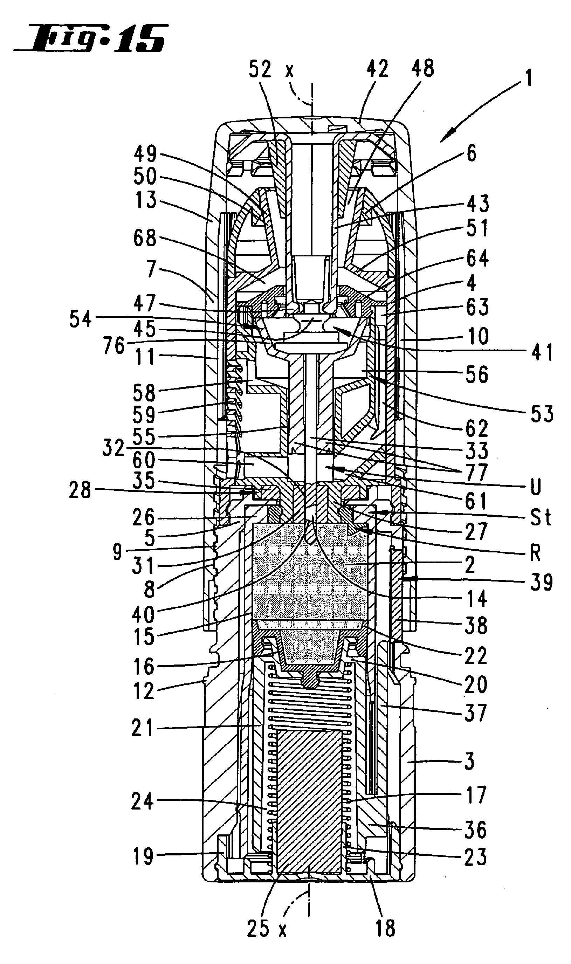

- Fig.15: eine Folgedarstellung der Fig. 14, eine Zwischenstellung betreffend;

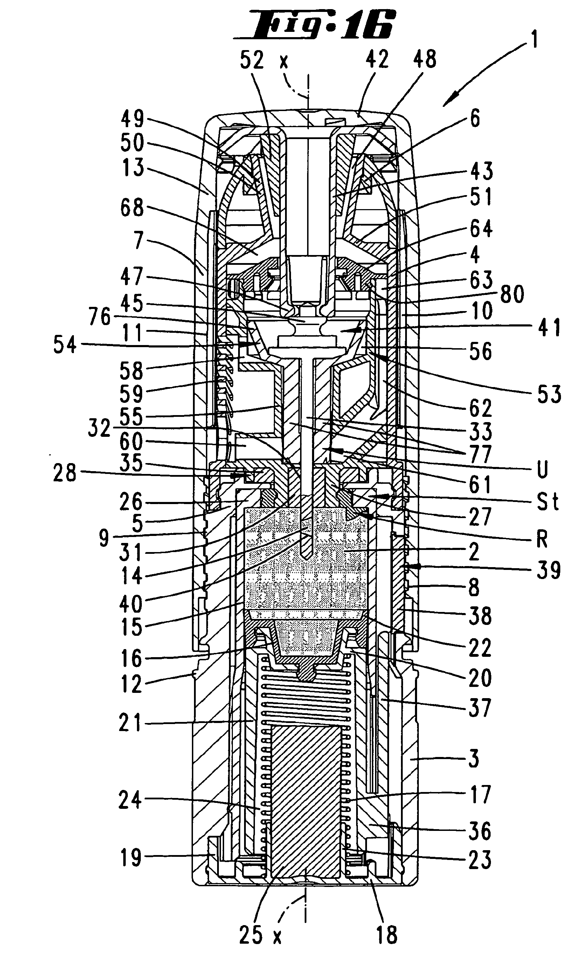

- Fig.16: eine Folgedarstellung der Fig. 15, eine Zwischenstellung im weiteren Verlauf des Aufschraubens der Verschlusskappe betreffend;
- Fig. 17: den Querschnitt durch die Dosiervorrichtung in der Entleerungs-Bereitschaftsstellung gemäß der Linie XVII - XVII in Fig. 8;
- Fig. 18: die Querschnittdarstellung durch die Dosiervorrichtung gemäß der Linie XVIII - XVIII in Fig. 11;
- Fig. 19: eine der Fig. 17 entsprechende Darstellung gemäß der Linie XIX - XIX in Fig. 11, die Entleerungs-Freigabestellung betreffend;
- Fig. 20: den Schnitt gemäß der Linie XX - XX in Fig. 11 durch die Vorratskammer unter Fortlassen der hier bevorrateten Substanz;
- Fig. 21: in einer perspektivischen Einzeldarstellung einen Innenzylinder der Dosiervorrichtung;
- Fig. 22: eine weitere perspektivische Darstellung des Innenzylinders;
- Fig. 23: den Dosierstab der Dosiervorrichtung in perspektivischer Einzeldarstellung;
- Fig. 24: eine perspektivische Einzeldarstellung des Kolbens;
- Fig. 25: in weiterer perspektivischer Einzeldarstellung einen rotorartigen Flügel zur Anordnung an dem Innenzylinder;
- Fig. 26: den rotorartigen Flügel in einer weiteren perspektivischen Darstellung;
- Fig. 27: in einer Einzeldarstellung die Unteransicht gegen einen Deckel einer Ringkammer.

Die in den Figuren dargestellte Dosiervorrichtung 1 zur Inhalierung einer pulverförmigen Substanz 2, insbesondere medizinischer Art, ist als bequem mitführbares, kurzstabförmiges Taschengerät realisiert, dies mit einem formbestimmenden zylindrischen Gehäuse 3.

Das zylindrische, rohrartige Gehäuse 3 besitzt kopfseitig einen relativ zum Gehäuse 3 um die Vorrichtungsachse x drehbaren Außenzylinder 4. Letzterer ist im Bereich einer endseitigen Radialstufe 5 an dem Gehäuse 3 rotierbar festgelegt.

Dieser gleichfalls zylindrische, rohrartige Außenzylinder 4 geht kopfseitig der Vorrichtung 1 in ein aufgesetztes Mundstück 6 über, welches mundgerecht ausgeformt ist, bspw. abgeflacht. Dieses Mundstück 6 ist mittels einer becherförmigen Verschlusskappe 7 schützend überfangbar. Letztere ist als Schraubkappe realisiert, wozu ein ihr zugeordnetes Innengewinde 8 in ein korrespondierendes Außengewinde 9 an der Mantelwand des Gehäuses 2 eingreift.

Der Außenzylinder 4 steht mit der Verschlusskappe 7 in drehfester Verbindung, wozu der Außenzylinder mantelwandaußenseitig vertikal ausgerichtete Rippen 10 aufweist, die mit entsprechend positionierten, schlitzartigen Vertikalnuten 11 wandungsinnenseitig der Verschlusskappe 7 zusammenwirken. Entsprechend löst eine Schraubbetätigung der Verschlusskappe 7 eine Rotation des Außenzylinders 4 um die Vorrichtungsachse x aus.

Fußseitig tritt der Stirnrand der becherförmigen Verschlusskappe 7 anschlagbegrenzend und über einen Konus dichtend gegen eine Ringschulter 12, welche aufgrund des vorgenannten Absatzes des zylindrischen Gehäuses 3 erzielt ist.

Die Verschlusskappe 7 dient zugleich als Betätigungshandhabe 13 zur Ausbringung der pulverförmigen Substanz 2 in reproduzierbaren Teilmengen 14, wozu der axiale Schraubhub des Gewindeeingriffs von Innengewinde 8 und Außengewinde 9 genutzt wird. Die Substanz 2 ist in einer Vorratskammer 15 des Gehäuses 3 (ggf. nachfüllbar) aufgenommen. Über eine Dosiervorrichtung wird jeweils eine Substanz-Teilmenge 14 an eine außerhalb der Vorratskammer 15 liegende Übergangsstelle U gefördert.

Bezüglich des dosierfähigen Gutes handelt es sich um eine (meist medizinische) pulverförmige Substanz 2. Es können beispielsweise saugstromtransportfähige Grundkörper wie Laktose Träger für oberflächenseitig anhaftende, mikronisierte Arzneimittel-Feinpartikel sein.

Den unteren Abschluss der Vorratskammer 15 bildet ein topfförmiger Druckboden 16, welcher in Richtung des Mundstückes 6 mittels einer Druckfeder 17 unter Federbelastung steht. Die Druckfeder 17 stützt sich mit der fußseitigen Endwindung an einer das Gehäuse 3 dort schließenden Bodenkappe 18 ab. Diese steht in Rasteingriff zum hier wandungsinnenseitig querschnittsgrößeren Abschnitt des Gehäuses 3, wobei ein entsprechender Rastkragen 19 der Bodenkappe 18 in eine passende Ringnut des Gehäuses 3 eingreift.

Die kopfseitige Endwindung der vorgespannten Druckfeder 14 wirkt belastend auf eine Innenschulter 20 eines Hohlkolbens 21 der kolbenförmigen Einrichtung 16/21. Wie aus den Darstellungen zu erkennen, ist der gestuft topfförmige Druckboden 16 im Bereich der Innenschulter 20 mit dem Hohlkolben 21 rastverbunden.

Der Topfrand des Druckbodens 16 stellt eine Ringlippe 22, die aufgrund ihres gummielastischen Materials die Wandung der Vorratskammer 15 verlustfrei abstreift.

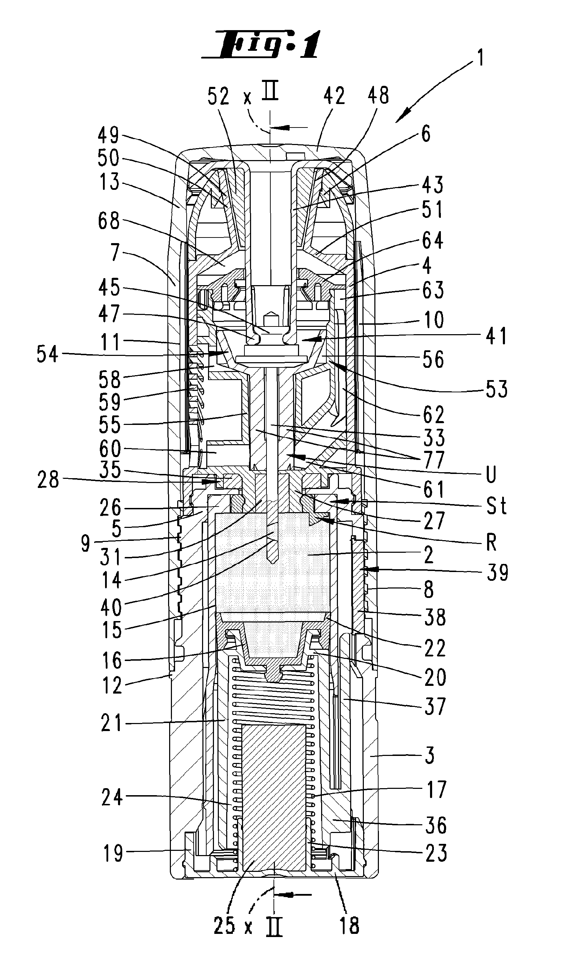

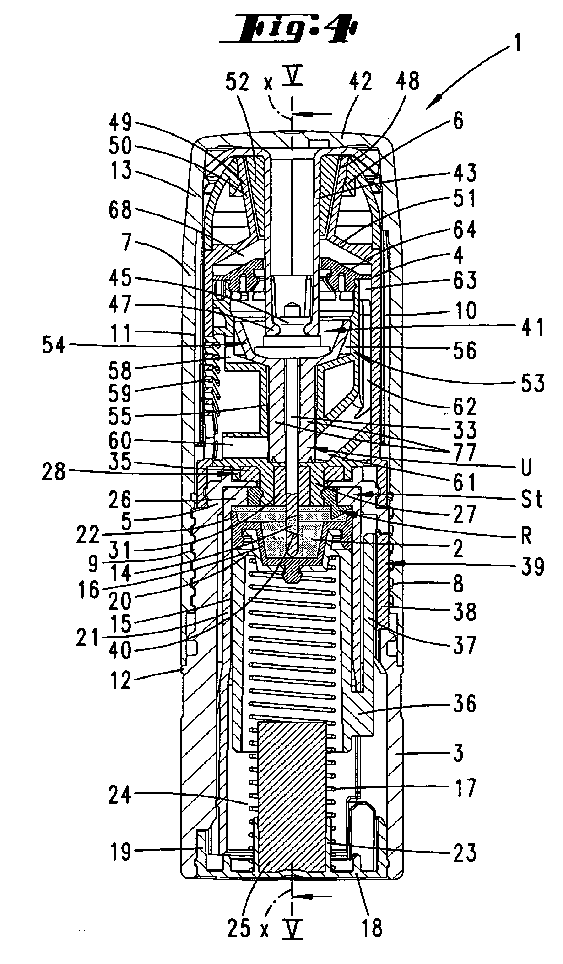

Die Druckfeder 17 ist in dem dargestellten Ausführungsbeispiel eine Zylinderfeder, mit einer im entspannten Zustand gemessenen Länge, die etwa einem Zehnfachen der maximalen Anpresslänge entspricht. Die Anpresslänge ist definiert durch das Axialverlagerungsmaß des Druckbodens 16 zwischen einer unteren, der Befüllstellung entsprechenden Stellung gemäß Figur 1 und einer oberen, anschlagbegrenzten Stellung des Druckbodens 16 in der Vorratskammer 15 gemäß Figur 4. So ist in dem dargestellten Ausführungsbeispiel eine solche Anpresslänge von 15 mm gegeben. Zufolge der Federausgestaltung, insbesondere zufolge der gewählten Federlänge ist über die gesamte Anpresslänge ein konstanter Federdruck auf den Druckboden 16 erreicht, was zu einer gleichmäßigen Substanzverdichtung über den gesamten Nutzungszeitraum der Vorrichtung 1 führt.

Von der Bodenkappe 18 geht zentral ein Hohl-Stehzapfen 23 aus. Dieser bildet zusammen mit dem diesen mit Abstand umgebenden Hohlkolben 21 eine Federkammer 24 für die Druckfeder 17. In dem hohlförmigen Stehzapfen 23 ist zentral ein Feuchtigkeit absorbierendes Material in Form einer Trockenmittelkapsel 25 gefasst. Übergangsseitig zum in Axialrichtung an dem Gehäuse 3 anschließenden Außenzylinder 4 schließt die Vorratskammer 15 mit einer einstückig mit der Mantelwand der Vorratskammer 15 ausgebildeten Kammerdecke 26 ab. Diese ist zentral durchsetzt von einem Zylinderabschnitt 27 eines sich in einer senkrechten Ebene zur Vorrichtungsachse x erstreckenden Drehteils 28.

Dieses ist im wesentlichen plattenförmiger Gestalt und ist drehfest mit dem Außenzylinder 4 verbunden, so entsprechend gegenüber der Kammerdecke 26 um die Vorrichtungsachse x drehbar. Der Zylinderabschnitt 27 erstreckt sich unterseitig des Drehteils 28, die Kammerdecke 26 durchsetzend. Die untere freie Stirnfläche des Zylinderabschnitts 27 liegt in der Ebene der die Vorratskammer 15 abdeckenden Fläche der Kammerdecke 26.

Die Durchbrechung in der Kammerdecke 26 ist gegenüber dem Durchmesser des Zylinderabschnitts 27 durchmesservergrößert. In dem verbleibenden Ring-spalt ist ein im Grundriss ringförmiger Halter für einen Rotor-Flügel R positioniert. Dieser ist drehfest mit dem Zylinderabschnitt 27 verbunden.

Die der Vorratskammer 15 zugewandte Innenfläche des Rotorrings 30 liegt in der Ebene der in entsprechender Richtung weisenden Stirnfläche des Zylinderabschnitts 27.

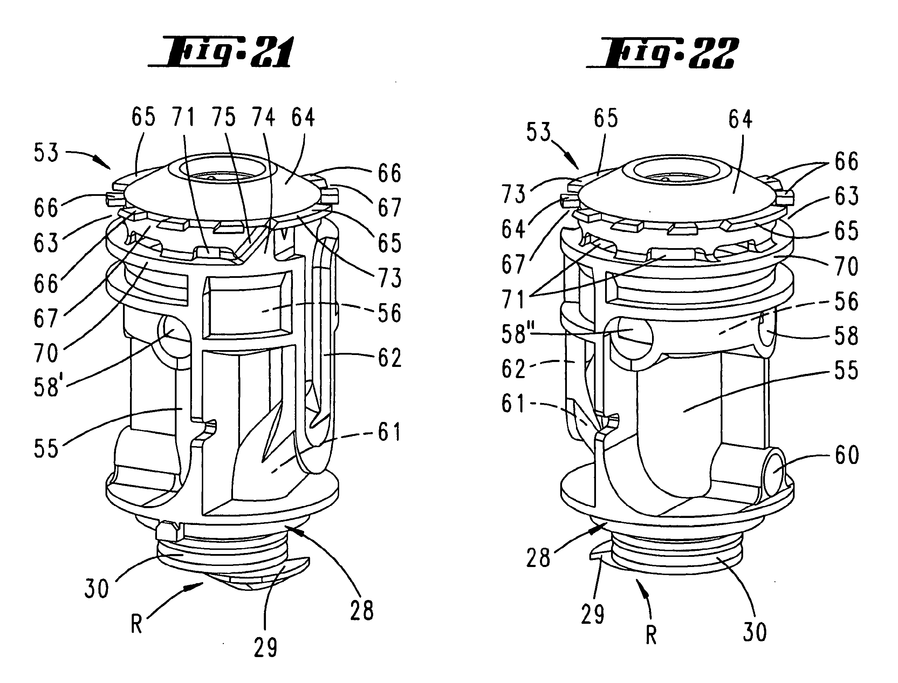

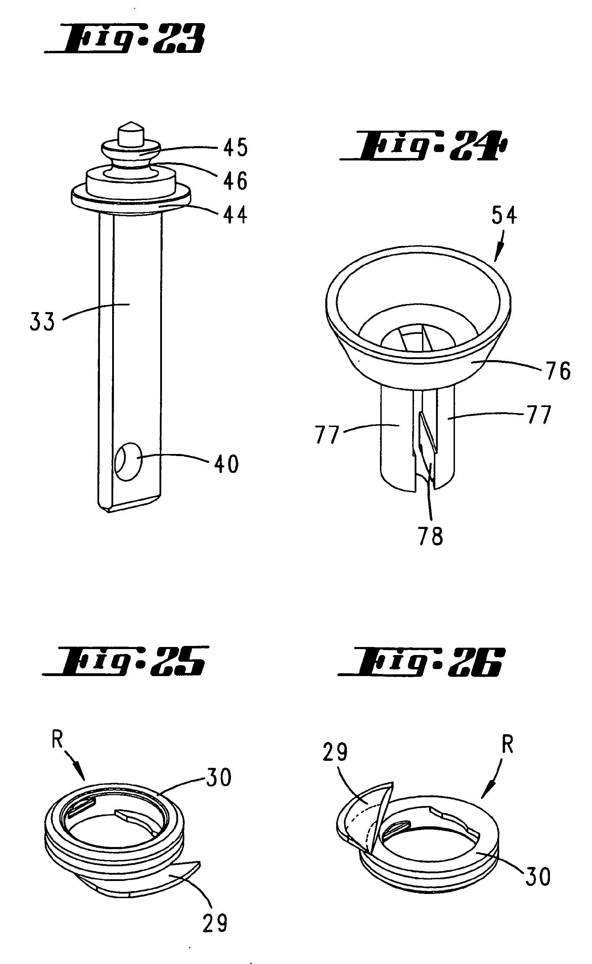

Der in den Figuren 22 und 23 in Einzeldarstellungen gezeigte Rotor R trägt unterseitig, das heißt der Vorratskammer 15 zugewandt, einen Flügel 29. Es handelt sich hierbei um einen kugelkappenabschruttförmigen Flügel 29, der radial über den Rotorring 30 des Rotors R nach außen übersteht. Der Flügel 29 unterfängt entsprechend den radial außerhalb des Rotors R anschließenden Bereich der Kammerdecke 26, dies bei ebener Ausgestaltung der der Kammerdecke 26 zugewandten Oberfläche des Flügels 29. Diese Fläche des Flügels 29 liegt an der zugewandten Kammerdeckenfläche an. In Radialerstreckung reicht der Flügel 29 bis zur Innenwandung der Vorratskammer 15. Von diesem radial äußeren Bereich steigt der Flügel 29 im Querschnitt nach radial innen konvex an, bis zu einer axialen Höhe, die etwa dem radialen Überstandsmaß des Flügels 29 über den Rotorring 30 entspricht.

Zufolge dieser Anordnung ragt der Flügel 29 des Rotors R in den Substanzvorrat der Vorratskammer 15 ein. Die durch die Kammerdecke 26 geformte Schulter bildet in Zusammenwirkung mit dem relativ zur Vorratskammer 15 drehbaren Rotor R bzw. Flügel 29 einen Stator St.

Der Rotor R ist über den Rotorring 30 an dem Zylinderabschnitt 27 des Drehteils 28 angeclipst.

Im Zentrum nimmt der Zylinderabschnitt 27 eine Dichtbuchse 31 auf. Diese besteht aus einem Gummimaterial oder ähnlichem elastischen Material. Diese belässt zentral eine im Querschnitt schlitzförmige Führungsöffnung 32 für einen querschnittsangepassten Dosierstab 33.

Die Dichtbuchse 31, wie auch weiter eine zwischen dem Drehteil 28 und einem gehäuseseitigen, die Kammerdecke 26 überfangenden Gehäuseabschnitt 34 vorgesehene Ringdichtung 35 können in einfachster Ausgestaltung im ZweiKomponenten-Spritzverfahren zusammen mit dem Drehteil 28 und darüber hinaus mit einem noch näher beschriebenen Innenzylinder gefertigt sein. Es ist jedoch diesbezüglich auch eine nachträgliche Anordnung der Gummi- bzw. Elastomerteile im Zuge der Herstellung möglich.

Der mit dem Druckboden 16 rastverbundene Hohlkolben 21 weist fußseitig einen Radialausleger 36 auf. An diesem ist ein die Vorratskammerwandung wandungsaußenseitig übergreifender, axial ausgerichteter Anzeigevorsprung 37 angeformt. Dessen in Abhängigkeit von der Druckbodenstellung erreichte Axialstellung ist von außen für den Benutzer durch ein in dem Gehäuse vorgesehenes Sichtfenster 38 erkennbar. Es ist hierdurch eine Füllstandsanzeige 39 gegeben.

Der Dosierstab 33 fungiert zufolge entsprechender Ausgestaltung als eine bewegte Dosierkammer 40 für die auszugebende Substanz-Teilmenge 14, wobei die Bewegung des Dosierstabes 33 linear in der Längsmittelachse x - x der im wesentlichen rotationssymmetrisch gestalteten Vorrichtung 1 erfolgt, überlagert von einer um die Längsmittelachse x - x durchgeführten Rotationsbewegung. Der Dosierstab 33 ist im wesentlichen als Flachteil ausgeformt mit langgestreckt rechteckigem Querschnitt. Das Längenverhältnis von Schmalseite zu Breitseite beträgt in dem dargestellten Ausführungsbeispiel etwa 1 : 3.

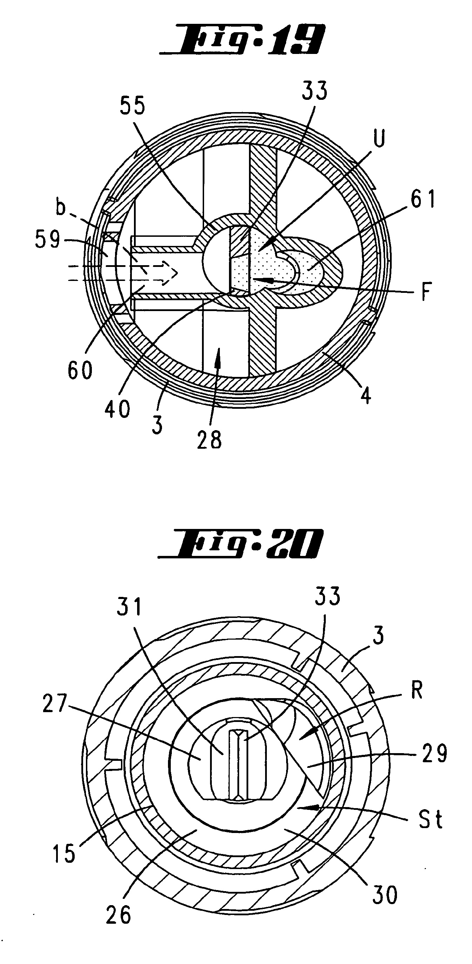

An dem dem Mundstück 6 abgewandten Ende bildet der Dosierstab 33 eine annähernd kreuzschlitzartige Zuspitzung aus. Die zwei spiegelsymmetrischen Schrägflanken gehen hierbei von den jeweiligen Breitseiten des Dosierstabes 33 aus (vgl. Figur 20).

Die Querschnittsausgestaltung des Dosierstabes 33 und die Ausspitzung des freien Endbereichs haben aufgrund der Drehmitnahme des Dosierstabes 33 im Zentrumsbereich auflockernd verdrängende Wirkung in Bezug auf den See aus pulverförmiger Substanz 2.

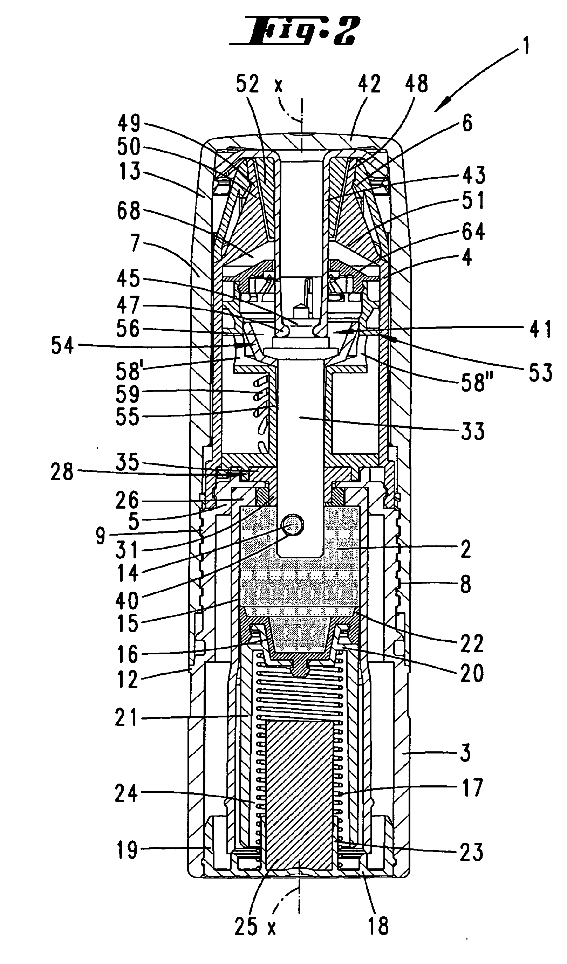

Die Dosierkammer 40 ist als im wesentlichen senkrecht zur Längsmittelachse x - x verlaufende Querbohrung realisiert, dies mit einer Bohrungsachse, die die Breitseitenflächen des Dosierstabes 33 durchsetzt. Die Querbohrung ist konisch geformt, so dass sich die Querbohrung zu einer Breitseitenfläche des Dosierstabes 33 hin verjüngt. Weiter ist, wie beispielsweise aus der Darstellung in Figur 2 zu erkennen, die im Bereich des in den Substanz-See einragenden Endes des Dosierstabes 33 ausgebildete Dosierkammer 40 mit Bezug auf die Breitseitenflächen des Dosierstabes 33 außermittig, das heißt seitlich versetzt zur Längsachse x - x angeordnet.

Der Hubweg der linear sowie überlagert rotatorisch bewegten Dosierkammer 40 berücksichtigt in beiden Endstellungen des Dosierstabes 33 ein Zuhalten des Querschnitts der Führungsöffnung 32 mit dosierkammerfüllender Rakel- bzw. Abstreichwirkung über die Länge der besagten Öffnung 33.

Das mundstückseitige Ende der Verschlusskappe 7 bildet eine bei Überlast ausklinkende Andockstelle 41 zwischen Dosierstab 33 und Verschlusskappe 7. Das verschlusskappenseitige Rastmittel ist dabei ein ausfederfähiger Hakenkranz, der im Bereich des freien Endes eines zentral unterseitig einer Verschlusskappendecke 42 angeordneten Hohlzylinders 43 ausgeformt ist. Das korrespondierende Ende des Dosierstabes 33 ist im Querschnitt rotationssymmetrisch ausgeformt, wobei weiter im Übergangsbereich vom Flachteilabschnitt zum zylinderförmigen Endabschnitt ein tellerförmiger Radialkragen 44 auswächst. Mit axialem Abstand zu diesem Radialkragen 44 formt der dem Flachteil abgewandte Endbereich des Dosierstabes 33 einen Rastkopf 45 aus. Zwischen diesem und dem Radialkragen 44 ist eine wespentaillenartige Ringnut 46 gebildet. In diese greifen einwärts gerichtete Nasen 47 der federnden Zungen des Hakenkranzes ein. Der Rastkopf 45 ist in beiden Axialrichtungen durch die Nasen 47 überwindbar. Die Verrastung kann recht stramm sein, weil sie bei der Schraubverlagerung der Kappe gelöst und wieder verbunden wird.

Die zentrale Öffnung 48 des Mundstückes 6 ist im Bereich eines Dispergierteils 49 ausgebildet. Dieses Dispergierteil 49 öffnet sich nach außen, das heißt abgewandt der Vorratskammer 15 konisch, wobei deren Wandung 50 zugewandt der Vorratskammer 15 übergeht in einen ringförmigen, dachartigen Deckenabschnitt 51. Dieser bildet zugleich den oberen Abschluss des das Mundstück 6 tragenden Außenzylinders 4.

Der von dem Dispergierteil 49 geschaffene zentrale Freiraum ist in der Kappenverschlussstellung zentral durchsetzt von dem die Nasen 47 tragenden Hohlzylinder 43. Der sich hierbei einstellende Ringraum zwischen Hohlzylinder 43 und Dispergierteil-Wandung ist in der Kappenverschlussstellung ausgefüllt durch eine weitere Trockenmittelkapsel 52.

In dem Außenzylinder 4 ist zentral von dem Dosierstab 33 und in der Kappenverschlussstellung vom verschlusskappenseitigen Hohlzylinder 43 durchsetzt ein Innenzylinder 53 aufgenommen. Dieser ist drehfest mit dem Außenzylinder 4 verbunden.

Dieser Innenzylinder 53 ist im wesentlichen als Hohlkörper gestaltet und trägt zentral einen in Axialrichtung verlagerbaren Kolben 54. Die Führung des Kolbens 54 ist etwa in der unteren Hälfte des Innenzylinders 53, zugewandt der Vorratskammer 15 durch einen im Querschnitt kreisrunden Führungsabschnitt 55 realisiert.

Der der Vorratskammer 15 abgewandte Abschnitt des Innenzylinders 53 formt einen gegenüber dem Führungsabschnitt 55 querschnittsvergrößerten Kolbenkopf-Verlagerungsbereich 56 aus, dessen axial ausgerichtete Bereichswandung 57 Radialöffnungen 58, 58' und 58" aufweisen. Diese Radialöffnungen stehen mit einem außenzylinderseitigen Gitterwandabschnitt 59 strömungsmäßig in Verbindung.

Unterhalb des Gitterwandabschnittes 59, weiter fußseitig des innenzylinderseitigen Führungsabschnittes 55 ist ein radial ausgerichteter, sich gleichfalls zum Gitterwandabschnitt 59 öffnender Strömungskanal 60 ausgebildet. Dieser kann auch als Sichtkontrollfenster gegen den Dosierstab 33 dienen. Er mündet in den vom Führungsabschnitt 55 zentral belassenen Freiraum. Radial gegenüberliegend des Strömungskanals 60 schließt sich an den Führungsabschnitt 55 ein Kanalzwischenabschnitt 61 an, der ausgehend von dem Führungsabschnitt 55 unter Einschluss eines Winkels von 45° zu einer senkrecht zur Achse x ausgerichteten Ebene in Richtung auf die zugeordnete Wandung des Außenzylinders 4 ansteigend verläuft, um dann endseitig in einen axial gerichteten Kanal 62 überzugehen. Dieser Kanal 62 ist durch eine axial ausgerichtete, schlitzartige, nach radial außen sich öffnende Ausnehmung in dem Innenzylinder-Mantel geschaffen. Die radiale Überdeckung des Kanals 62 ist erreicht durch die zugeordnete Wandung des Außenzylinders 4.

Neben der beispielsweise in der Schnittdarstellung in Figur 1 zu erkennenden Radialöffnung 58 sind zwei weitere, jeweils in einer quer zur Achse x ausgerichteten Ebene betrachtet einen Winkel von 90° zu dieser Radialöffnung 58 einschließende weitere Radialöffnungen 58' und 58" vorgesehen, die durch entsprechende Ausgestaltung der Innenzylinder-Wandung in direktem Luftströmungsanschluss stehen mit dem Gitterwandabschnitt 59.

Der axial ausgerichtete Kanal 62 mündet mit seinem dem Mundstück 6 zugewandten Ende in eine Ringkammer 63. Diese formt eine Wirbelkammer aus. Deren Decke 64 ist im Querschnitt dachartig gestaltet und ist mit randseitig ausladenden, vorstehenden Flügeln 65, 66 ausgestattet. Diese treten randseitig gegen die Innenwandung des Außenzylinders 4 und belassen in Umfangsrichtung betrachtet zwischen sich Zwischenräume 67, durch welche ein Luftströmungsanschluss zwischen Ringkammer 63 und einem zwischen Dispergierteil-Deckenabschnitt 51 und Ringkammerdecke 64 belassenen weiteren Ringraum 68 erreicht ist.

Die Decke 64 ist mit einem axial gerichteten Flansch 69 wandungsinnenseitig an dem Innenzylinder 53 festgelegt.

Der Ringkammerboden 63 ist gebildet durch einen mit axialem Abstand zu den Flügeln 65, 66 der Decke 64 wandungsaußenseitig am Innenzylinder 53 radial nach außen abragenden Ringkragen 70. Auch dieser stützt sich randseitig wandungsinnenseitig des Außenzylinders 4 ab. Dieser Ringkragen 70 ist von dem axial ausgerichteten Kanal 62 durchbrochen. Nach radial innen ist die Ringkammer 63 begrenzt durch einen endseitigen, der Verrastung der Decke 64 dienenden Wandungsabschnitt des Innenzylinders 53. Die so gebildete Ringkammerwandung ist mit schlitzartigen Durchbrüchen 71 versehen, zum luftströmungsmäßigen Anschluss der Ringkammer 63 mit dem Kolbenkopf-Verlagerungsbereich 56.

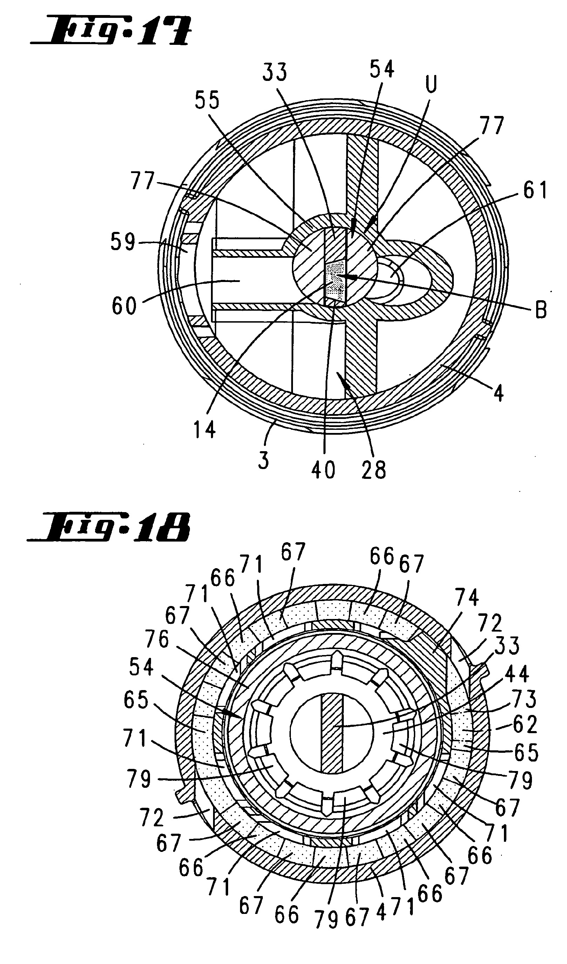

Wie weiter insbesondere aus der Schnittdarstellung in Figur 18 zu erkennen, ist die Außenzylinderwandung auf Höhe der Ringkammer 63 mit zwei diametral gegenüberliegend angeordneten Lufteintrittsöffnungen 72 versehen. Diese münden tangential gerichtet in die Ringkammer 63, dies weiter unter Vorgabe einer gemeinsamen Strömungsrichtung. Entsprechend wird durch Einsaugen durch die Lufteintrittsöffnungen 72 eine vorgegebene Luftströmung in der Ringkammer 63 erreicht. Der axial ausgerichtete Kanal 62 mündet in Strömungsrichtung betrachtet unmittelbar hinter der Mündung einer Lufteintrittsöffnung 72 in der Ringkammer 63, so dass der durch den axialen Kanal 62 in die Ringkammer 63 eintretende Luftstrom über die Lufteintrittsöffnungen 72 eine gezielte Umlenkung in die gewünschte Wirbelrichtung erfährt.

Die Flügel der Decke 64 sind in Umfangsrichtung betrachtet unterschiedlich breit gestaltet. So sind zwei diametral gegenüberliegende Flügel 65 gegenüber den weiteren Flügeln 66 in Umfangsrichtung betrachtet mit einem etwa dreifachen Breitenmaß versehen. Einer dieser verbreiterten Flügel 65 liegt in Überdeckung zum Mündungsbereich des axialen Kanals 62 in die Ringkammer 63, formt demzufolge einen Umlenk-Abprallwandflügel 73 für den durch den axialen Kanal 62 in die Ringkammer 63 eintretenden Saugluftstrom.

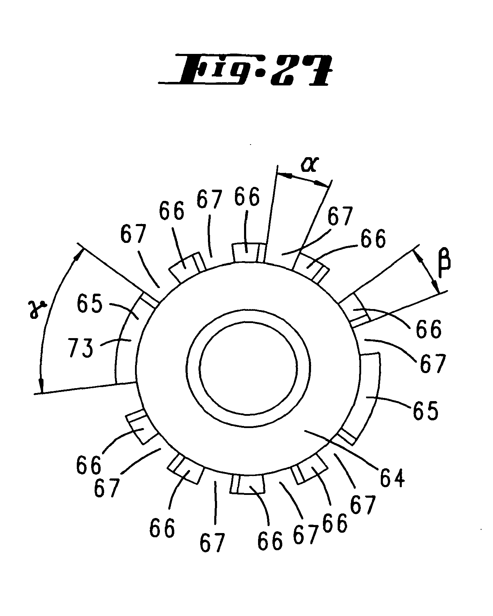

Wie weiter insbesondere aus der Einzeldarstellung in Figur 27 zu erkennen, erstrecken sich die Flügel 66 in dem beschriebenen Ausführungsbeispiel in Umfangsrichtung über einen Winkel β von 15°. Die zwischen den Flügeln 66 und 65 belassenen Zwischenräume 67 erstrecken sich in Umfangsrichtung gleichfalls über einen Winkel α von 15°, während die Randkanten der breiteren Flügel 65 einen Winkel δ von 45° einschließen.

Es sind diesbezüglich auch andere Aufteilungen möglich (bspw. kleinere Flügel - größere Zwischenräume; größere Flügel - kleinere Zwischenräume; unregelmäßige Ausgestaltung von Flügel und Zwischenräumen).

In der durch die Anordnung der Lufteintrittsöffnungen 72 entgegengesetzten Luftströmungsrichtung in der Ringkammer 63 ist benachbart zur Mündung des axialen Kanals 62 in der Ringkammer 63 ein Unterbrecher 74 angeordnet. Dieser begrenzt den Umfangsweg der Ringkammer 63, der entsprechend zufolge dieser Ausgestaltung nicht durchgehend ringförmig, sondern vielmehr unterbrochen ausgebildet ist. Die entgegen der Strömungsrichtung weisende Rückflanke des Unterbrechers 74 stellt eine Auflaufschräge 75 dar, verbindend den Ringkammerboden mit der die Zwischenräume 67 aufweisenden Ringkammerdecke. Es ist so eine Zwangsauslenkung des Luftstromes im Endbereich der Ringkammer 63 nach axial oben in den weiteren Ringraum 68 erreicht.

Der in dem Innenzylinder 53 drehfest gehaltene, aber axial verlagerbare Kolben 54 weist zunächst einen tellerförmig sich in Richtung auf das Mundstück öffnenden Kolbenkopf 76 auf. Dieser öffnet sich im Querschnitt konusartig. Unterseitig des Kolbentellers sind zwei parallel zueinander verlaufende, axial ausgerichtete Zungen 77 angeformt. Der Kolben 54 besteht aus einem gummiartigen Material.

Die wandungsaußenseitig die Querschnittskontur des Führungsabschnittes 55 des Innenzylinders 53 aufnehmenden Zungen 77 sind an ihrem unteren freien

Rand lippenartig gespalten, besitzen weiter in ihrem freien Randbereich materialverstärkte Abdichtungsflächen 78.

Zwischen den Zungen 77 ist das Flachteil des Dosierstabes 33 geführt, wobei die Abdichtungsflächen 78 in Zusammenwirkung mit dem Flachteil des Dosierstabes 33 abstreifende und abdichtende Wirkung haben.

In einer Vorrichtungsgrundstellung gemäß der Darstellung in Figur 1 treten die lippenartig gespalteten freien Ränder der Zungen 77 innerhalb einer Axialvertiefung oberseitig gegen den Zylinderabschnitt 27.

Weiter liegt in dieser Grundstellung der tellerartige Kolbenkopf 76 auf einem Bodenbereich des Kolbenkopf-Verlagerungsbereiches 56 anschlagbegrenzt auf. Der umlaufende Randbereich des freien Endes des Kolbenkopfes 76 liegt dichtend gegen die zugeordnete Innenwandung des Innenzylinders 53 an.

Weiter liegt in dieser Grundstellung der Kopf des Dosierstabes 33, das heißt dessen Radialkragen 44 und Rastkopf 45 in der durch die tellerartige Ausgestaltung des Kolbenkopfes 76 geschaffenen Vertiefung ein.

Der Kolbenkopf 76 liegt hierbei mit axialem Abstand unterhalb der Decke 64.

### Die Funktionsweise der angegebenen Vorrichtung 1 ist wie folgt:

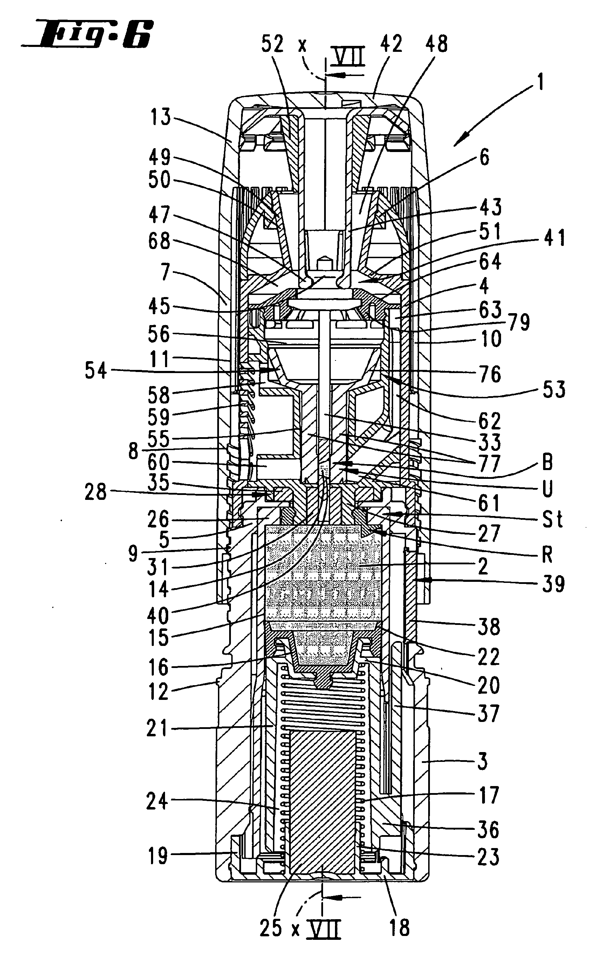

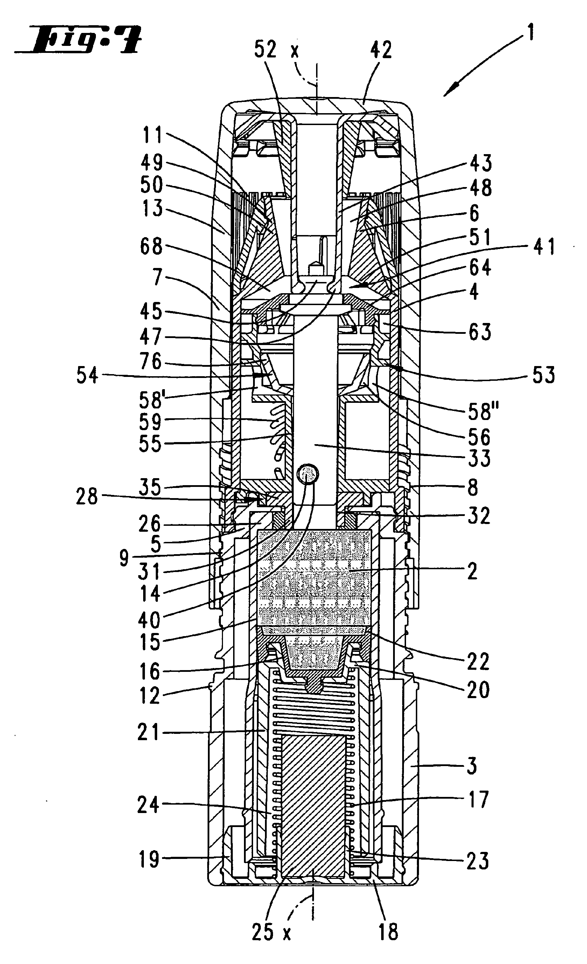

Zur Vorbereitung der Inhalation ist zunächst die Verschlusskappe 7 abzudrehen. Im Zuge des Schraubabhebens der Verschlusskappe 7 folgt über die angegebene Kupplung eine Drehmitnahme des Außenzylinders 4 und über diesen des Innenzylinders 53, weiter in dem angegebenen Ausführungsbeispiel alle Teile oberhalb der Vorratskammerebene, die nicht drehfest mit dem Gehäuse 3 verbunden sind. Entsprechend wird auch der Dosierstab 33 drehmitgeschleppt, wobei weiter durch die schraubabhebende Verlagerung der Verschlusskappe 7 zugleich eine axiale Verlagerung des Dosierstabes 33 über die Andockstelle 41 erfolgt, was eine schraubengangartige Verlagerung der Dosierkammer 40 in die in Überdeckung zu dem Strömungskanal 60 liegende, noch verschlossene Entleerungs-Bereitschaftsstellung B gemäß der Darstellung in den Figuren 6 und 7 bewirkt.

Durch die zur Rotationsachse des Dosierstabes 33 exzentrische Anordnung der Dosierkammer 40 ist eine optimale Befüllung derselben mittels schraubengangförmigen Durchtauchens durch den Substanz-See erreicht, unterstützt durch den Rotor. Hierbei weist die durchmessergrößere Öffnungsfläche der Dosierkammer 40 in Rotationsrichtung.

Der zugleich rotierende Flügel 29 des Rotors R bewirkt hierbei ein stets gelockertes Umfeld des Substanz-Sees, wobei ein Schaufeleffekt erreicht ist. Bei entgegengesetzter Rotation des Rotors R - beim Wiederaufschrauben der Verschlusskappe 7 - wirkt der Flügel 29 mit dem Stator St zusammen zum schabenden Abtragen von Substanz 2 von der statorseitigen Fläche und zum Niederdrücken der Substanz 2, womit eine Vergleichmäßigung des Substanz-Sees erreicht wird. Der Flügel 29 des Rotors R wirkt entsprechend in beiden Rotationsrichtungen auf den Substanz-See ein.

Mit Erreichen der Entnahme-Bereitschaftsstellung B des Dosierstabes 33 wird dieser rastend festgelegt. Hierzu fährt der Radialkragen 44 des Dosierstabes 33 hinter Rastfinger 79, die unterseitig an der Decke 64 ausgeformt sind.

Bei weiterer Schraubverlagerung der Verschlusskappe 7 wird die Verrastung im Bereich der Andockstelle 41 zwischen Hohlzylinder 43 und Dosierstab 33 aufgehoben. Die Nasen 47 verlassen entsprechend die Ringnut 46, wonach die Verschlusskappe 7 abnehmbar ist. Die Vorrichtung 1 ist nunmehr vorbereitet zur Inhalation.

Durch die Schraubverlagerung der Verschlusskappe 7 kann eine ausreichende Kraft zur Herstellung der Verrastung von Radialkragen 44 und Rastfinger 79 und weiter zur Aufhebung der Verrastung zwischen Rastkopf 45 und kappenseitigen Nasen 47 vorgesehen sein.

Die Zungen 77 des Kolbens 54 liegen beidseitig die Dosierkammer 40 überdeckend an. Entsprechend kann in dieser Stellung die Substanz-Teilmenge 14 auch nicht teilweise ausrieseln. Vielmehr ist diese sicher gefangen in der Dosierkammer 40. Hierdurch ist einer Doppeldosierung bei nicht durchgeführter Inhalation und abschließendem Verschluss über die Verschlusskappe 7 entgegengewirkt. Weiter kann die Vorrichtung 1 in der Entnahme-Bereitschaftsstellung B der Dosierkammer 40 auch beiseite gelegt werden. Selbst übliche Stöße gegen die Vorrichtung 1 führen nicht zum Ausrieseln der zu inhalierenden Substanz-Teilmenge 14, was das Inhalationsergebnis verfälschen würde.

Der Inhalationsvorgang erfolgt selbstauslösend durch Saugluftbeaufschlagung durch den Benutzer, im weiteren in einfachster Weise durch Einatmen.

Über das Mundstück 6 wird Luft angesaugt, was zunächst durch Luftbeaufschlagung des Kolbenkopfes 76 zu einer Axialverlagerung des Kolbens 54 in Richtung auf die Decke 64 bewirkt. Der Auslösedruck liegt bei dem dargestellten Ausführungsbeispiel bei etwa 2 kgPa. Die Auslösung erfolgt weitestgehend schlagartig.

Der obere freie Randbereich des Kolbenkopfes 76 tritt in der angehobenen Stellung unterseitig gegen eine Ringwand 80 der Decke 64. Der nunmehr den freien Randbereich des Kolbenkopfes 76 umgebende Ringraum des Innenzylinders 53 ist radial erweitert, womit der Kolben 54 im Bereich des Kolbenkopfes 76 radial umströmt wird. Es ergibt sich hieraus ein Hauptluftstrom a, der durch den Gitterwandabschnitt 59, die Radialöffnungen 58, 58' und 58" durchtretend in den Kolbenkopf-Verlagerungsbereich 56 einströmt und durch den radial außerhalb des Kolbenkopfes 76 belassenen Ringraumbereich durch die Öffnungen 71 in die Ringkammer 63 tritt. Über diesen Luftströmungsweg wird etwa 85 bis 90 % des gesamten Inhalier-Luftvolumens transportiert.

Zugleich wird über die stets offenen radialen Lufteintrittsöffnungen 72 unmittelbar Luft in die Ringkammer 63 eingesaugt, zur Vorgabe der Wirbelrichtung in der Ringkammer 63.

Durch den axial verlagerten Kolben 54 sind die Zungen 77 gleichfalls axial verlagert, zur Freigabe der Dosierkammer 40. Eine unterstützende Wirkung zur Axialverlagerung des Kolbens 54 ist dadurch erreicht, dass der die Zungen 77 aufnehmende Führungsabschnitt 55 sich in Richtung auf den Kolbenkopf 76 leicht erweitert, womit die Reibung zwischen den Zungen 77 und der Wandung des Führungsabschnittes 55 reduziert ist. Auch die Reibung zwischen den Zungen 44 und dem Flachteil des Dosierstabes 33 ist minimiert auf den Bereich der Abdichtungsflächen 78.

Die Dosierkammer 40 liegt hiernach in einer Entnahme-Freigabestellung F, in welcher diese im Strömungsweg zwischen Strömungskanal 60 und Kanalzwischenabschnitt 61 freigeschaltet ist. Über diesen Substanztransport-Luftstrom b wird in dem dargestellten Ausführungsbeispiel etwa 10 bis 15 % des Inhalier-Luftvolumens transportiert.

Die Dosierkammer wird unter Durchsaugen von Seiten des Strömungskanals 60 ausgeräumt, dies weiter ausgehend von der kleineren Öffnungsfläche in Richtung der größeren Öffnungsfläche der Dosierkammer 40. Die zweifache Umlenkung um je etwa 45° in den gewinkelten Kanalzwischenabschnitt 61 und von diesem in den axial ausgerichteten Kanal 62 führt in Art einer Prallplattenwirkung zu einem ersten Aufbrechen größerer Pulverpartikel, was weiter zu einem verbesserten Inhalierergebnis führt.

Der über den Kanal 62 axial in die Ringkammer 63 mit einer relativ hohen Geschwindigkeit einströmende, substanzbehaftete Luftstrom wird über den Umlenk-Abprallwandflügel 73 und unterstützt durch die Initialströmung seitens der radialen Lufteintrittsöffnungen 72 in Umlaufrichtung abgelenkt. An diesem Umlenk-Abprallwandflügel 73 erfolgt ein weiteres Aufbrechen größerer Pulverpartikel.

Zufolge dieser Ausgestaltung wird der substanzbehaftete Luftstrom außerhalb des Kolbenbereiches geführt. Der Kolben 54 wird lediglich mit pulverfreier Luft umströmt.

In der Ringkammer 63 wird eine optimale Verteilung der zu inhalierenden Substanz-Teilmenge 14 erreicht. Die substanzbehaftete Luft tritt durch die Zwischenräume 67 zur Inhalation aus. Relativ schwere, gegebenenfalls nicht oder nicht ausreichend aufgebrochene Pulverpartikel werden spätestens über den Unterbrecher 74 in den Ringraum 68 gelenkt.

In der Ringkammer 63 werden die zunächst im wesentlichen axial anströmenden Luftströme a und b in eine gemeinsame horizontale Umlaufrichtung gelenkt, um hiernach gemeinsam unter axialer Durchsetzung der Decke 64 in das Mundstück 6 zu treten.

Dem Benutzer sind mehrere Merkmale für eine erfolgreiche Inhalation gegeben. Zum Einen ist eine optische Kontrolle dadurch gegeben, dass der Kolben 54 nach einem saugluftbedingten Anheben in seiner angehobenen Stellung aufgrund der gegebenen, wenn auch geringen Reibkräfte gehalten ist. Durch den nach radial außen offenen Strömungskanal 60 ist der Kolben 54 bzw. dessen Zungen 77 in der Entnahme-Bereitschaftsstellung B zu sehen. Dies kann weiter unterstützt sein durch eine optisch auffallende Farbgebung der Zungen 77. Nach erfolgter Inhalation und entsprechend angehobenem Kolben 54 sind die Zungen 77 nicht zu sehen. Vielmehr ist ein freier Blick auf die leere Dosierkammer 40 gegeben. Auch das Anschlagen des Kolbens 54 unterseitig an die Decke 64 ist sowohl akustisch als auch haptisch erfassbar.

Nach erfolgter Inhalation, alternativ aber auch bei nicht gewünschter Inhalation aus der Entnahme-Bereitschaftsstellung B heraus, wird die Verschlusskappe 7 wieder aufgeschraubt, wobei zunächst durch mittels Beaufschlagung des Rastkopfes 45 durch die Nasen 47 die Verrastung zwischen Radialkragen 44 und den Rastfingern 79 aufgehoben wird. Die Haltekräfte dieser Rastverbindung sind entsprechend kleiner eingestellt als das Kräftemaß zum Auslenken der Nasen 47. Im Zuge der weiteren Schraubabwärtsverlagerung der Verschlusskappe 7 wird über den dosierstabseitigen Radialkragen 44 der Kolben 54 wieder zurück in seine Grundstellung verfahren. Gleichzeitig wird unter axialer Verlagerung und entsprechender Rotationsbewegung der Dosierstab 33 abwärts in die Vorratskammer verlagert. Die Schieberückverlagerung des Kolbens 54 über den Dosierstab 33 endet mit Anschlag der freien, auslippenden Enden der Zungen 77 an der zugewandten Deckenfläche des Zylinderteils 27. Bei weiterer Schraubabwärtsverlagerung treten abschließend die Nasen 47 in die Ringnut 46 des Dosierstabes 33 ein. Dieses abschließende Verrasten ist für den Benutzer akustisch und haptisch als vollendeter Schließvorgang wahrnehmbar. So ist auch sichergestellt, dass eine das Mitschleppen des Dosierstabes 33 und somit der Dosierkammer 40 in die Entnahme-Bereitschaftsstellung B bewirkende Verrastung zwischen Dosierstab 33 und Verschlusskappe 7 nur in der untersten Stellung des Dosierstabes 33 erreicht wird, in welcher Stellung die Befüllung der Dosierkammer 40 vorgenommen wird. Entsprechend wird beim Anheben des Dosierstabes 33 immer eine gefüllte Dosierkammer 40 bereitgestellt.

Einer Fehlbedienung ist sicher entgegengewirkt. Ein nicht ordnungsgemäßes Verschließen der Vorrichtung 1 führt beim nächsten Inhalierversuch dazu, dass der entsprechend nicht angehobene Dosierstab 33 zum Einen mit seinem Flachteilabschnitt den Durchgang zwischen Strömungskanal 60 und Kanalzwischenabschnitt 61 verschließt. Zum Anderen beaufschlagt der Dosierstab 33 über den Radialkragen 44 weiter die zugeordnete Fläche des Kolbenkopfes 76. Entsprechend kann bei einem Inhalationsversuch bedingt durch den Verschluss des Strömungskanals 60 und die Blockade des Kolbens 54 keine Luftströmung aufgebaut werden (mit Ausnahme der geringen Strömung über die kleinen radialen Lufteintrittsöffnungen 72). Dem Benutzer ist ein klares Signal damit gegeben, dass eine Fehlstellung vorliegt. Diese kann nur durch ordnungsgemäßes Schließen der Vorrichtung 1 behoben werden.

Alle offenbarten Merkmale sind (für sich) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

### Bezugszeichenliste

- 1: Vorrichtung
- 2: Substanz
- 3: Gehäuse
- 4: Außenzylinder
- 5: Radialstufe
- 6: Mundstück
- 7: Verschlusskappe
- 8: Innengewinde
- 9: Außengewinde
- 10: Rippen
- 11: Nuten
- 12: Ringschulter
- 13: Betätigungshandhabe
- 14: Substanz-Teilmenge
- 15: Vorratskammer
- 16: Druckboden
- 17: Druckfeder
- 18: Bodenkappe
- 19: Rastkragen
- 20: Innenschulter
- 21: Hohlkolben
- 22: Ringlippe
- 23: Stehzapfen
- 24: Federkammer
- 25: Trockenmittelkapsel
- 26: Kammerdecke
- 27: Zylinderabschnitt
- 28: Drehteil
- 29: Flügel
- 30: Rotorring
- 31: Dichtbuchse
- 32: Führungsöffnung
- 33: Dosierstab
- 34: Gehäuseabschnitt
- 35: Ringdichtung
- 36: Radialausleger
- 37: Anzeigevorsprung
- 38: Sichtfenster
- 39: Füllstandsanzeige
- 40: Dosierkammer
- 41: Andockstelle
- 42: Verschlusskappendecke
- 43: Hohlzylinder
- 44: Radialkragen
- 45: Rastkopf
- 46: Ringnut
- 47: Nasen
- 48: Mundstück-Öffnung
- 49: Dispergierteil
- 50: Wandung
- 51: Deckenabschnitt
- 52: Trockenmittelkapsel
- 53: Innenzylinder
- 54: Kolben
- 55: Führungsabschnitt
- 56: Kolbenkopf-Verlagerungsbereich
- 57: Bereichswandung
- 58: Radialöffnung
- 58': Radialöffnung
- 58": Radialöffnung
- 59: Gitterwandabschnitt
- 60: Strömungskanal
- 61: Kanalzwischenabschnitt
- 62: Kanal
- 63: Ringkammer
- 64: Decke
- 65: Flügel
- 66: Flügel
- 67: Zwischenräume
- 68: Ringraum
- 69: Flansch
- 70: Ringkragen
- 71: Öffnungen
- 72: Lufteintrittsöffnungen
- 73: Umlenk-Abprallwandflügel
- 74: Unterbrecher
- 75: Auflaufschräge
- 76: Kolbenkopf
- 77: Zungen
- 78: Abdichtungsflächen
- 79: Rastfinger
- 80: Ringwand

- x: Vorrichtungsachse

- B: Entnahme-Bereitschaftsstellung
- F: Entnahme-Freigabestellung
- R: Rotor

- St: Stator
- U: Übergangsstelle

- α: Winkel Zwischenräume 67
- β: Winkel Flügel 66
- δ: Winkel Flügel 65

- a: Hauptluftstrom
- b: Substanztransport-Luftstrom

## Patentansprüche

1. Vom Saugluftstrom des Benutzers aktivierbare Dosiervorrichtung (1) zur Inhalierung einer pulverförmigen Substanz (2), insbesondere medizinischer Art, die in einer Vorratskammer (15) angeordnet und aus dieser mittels einer Dosierkammer (40) eines Dosierstabes (33) in eine Entleerungs-Bereitschaftsstellung (B) bringbar ist, in welcher Stellung die Dosierkammer (40) von einem Kolben (54) verschlossen ist, welch letzterer mittels des Benutzer-Saugluftstromes in Richtung eines Mundstückes (6) in eine Entlee-rungs-Freigabestellung verlagerbar ist, **dadurch gekennzeichnet, dass** der im oberen Bereich tellerförmig gestaltete Kolben (54) mit von der Tellerunterseite ausgehenden, die Dosierkammer (40) - oder mehrere Dosierkammern - in der Entleerungs-Bereitschaftsstellung verschließenden Zungen (77) ausgestattet ist, welche bei der vom Benutzer-Saugluftstrom veranlassten Kolbenverlagerung die Dosierkammer (40) freigeben/öffnen.

2. Dosiervorrichtung nach Anspruch 1, **dadurch gekennzeichnet, dass** der obere Rand des Kolbens (54) in seiner oberen Endstellung vor eine Ringwand (80) tritt, die zu einer Ringkammer (63) gehört, welche Ringkammer (63) mit randseitig ausladenden Flügeln (65, 66) ausgestattet ist, welche zwischen sich Zwischenräume (67) belassen, und dass oberhalb der Decke (64) der Ringkammer (63) eine geneigt stehende Prallwand (51) vorgesehen ist.

3. Dosiervorrichtung nach Anspruch 2, **dadurch gekennzeichnet, dass** ein Teil der Flügel (65) in Umfangsrichtung breiter gestaltet ist zur Bildung eines Umlenk-Abprallwandflügels (73) für den Saugluftstrom.

4. Dosiervorrichtung nach Anspruch 2, **dadurch gekennzeichnet, dass** ein Rastkopf (45) des Dosierstabes (33) jedenfalls teilweise in eine obere Vertiefung des Kolbens (54) eingesenkt ist, wobei der Kolben ein Tellerkolben ist.

5. Dosiervorrichtung nach Anspruch 3, **dadurch gekennzeichnet, dass** man-telwand-seitig eines von einer Verschlusskappe (7) bewegten Innenzylinders H (53) ein axial verlaufender Kanal (62) vorgesehen ist, der von der Entleerungsseite der Dosierkammer (40) ausgeht und in der Ringkammer (63) endet, wobei zur Umlenkung der axialen Luftstrom-Richtung in die Umlaufebene der Umlenk-Abprallwandflügel (73) vorgesehen ist.

6. Dosiervorrichtung nach Anspruch 1, **dadurch gekennzeichnet, dass** die Zungen (77) an ihrem unteren freien Rand lippenartig gespalten sind zur Erzielung einer Klemmwirkung, und dass die Zungen (77) materialverstärkte Abdichtungsflächen (78) besitzen.

7. Dosiervorrichtung nach Anspruch 1, **dadurch gekennzeichnet, dass** fluchtend zur Dosierkammer (40) - in verschlossener Entleerungs-Bereitschaftsstellung - ein Kanal (60) auf die eine Zunge (77) gerichtet ist, insbesondere zur optischen Kontrollmöglichkeit.

8. Dosiervorrichtung nach Anspruch 1, **gekennzeichnet durch** zwei Luftströmungen (a, b), von denen der eine die Entleerungs-Bereitschaftsstellung (B) der Dosierkammer (40) öffnet und der zweite direkt in eine dem Mundstück (6) vorgeschaltete Ringkammer (63) führt, wo sich beide Luftströme treffen.

9. Dosiervorrichtung nach Anspruch 8, **dadurch gekennzeichnet, dass** eine Lufteintritts-Gitterfläche an einem Außenzylinder (4) auf derjenigen Seite des Dosierstabes (33) liegt, welche der Entleerungsrichtung der Dosierkammer (40) gegenüberliegt und dass unterhalb der Lufteintritts-Gitterfläche auf Höhe der von der Dosierkammer (40) in der Entleerungs-Bereitschäftsstellung (B) eingenommenen Position ein auf die Dosierkammer (40) gerichteter Strömungskanal (60) angeordnet ist.

10. Dosiervorrichtung nach Anspruch 9, **dadurch gekennzeichnet, dass** ein Innenraum eines Innenzylinders (53) voll zur freien Verteilung für die durch die Lufteintritts-Gitterfläche angesogene Luft zur Verfügung steht und mit der Ringkammer (63) in Strömungsverbindung liegt.

11. Dosiervorrichtung nach Anspruch 9, **dadurch gekennzeichnet, dass** die Mantelwand des Außenzylinders (4) mindestens eine, vorzugsweise zwei radial gegenüberliegende Lufteintrittsöffnungen (72) besitzt und die Lufteintrittsöffnungen (72) unter Vorgabe einer gemeinsamen Strömungsrichtung tangential gerichtet in die Ringkammer (63) münden.

12. Dosiervorrichtung nach Anspruch 1, **gekennzeichnet durch** eine dem echten Füllstand zugeordnete Anzeige (39) im Bereich der Vorratskammerwänd.

13. Dosiervorrichtung nach Anspruch 1, **dadurch gekennzeichnet, dass** die Aufwärtsbewegung eines Dosierkammer-Kolbens (16) gestoppt ist.

14. Dosiervorrichtung nach Anspruch 1, **dadurch gekennzeichnet, dass** der Dosierstab (33) in seiner Hochstellung auslösbar verrastet.

15. Dosiervorrichtung nach Anspruch 14, **dadurch gekennzeichnet, dass** ein Radialkragen des Dosierstabes (33) hinter Rastfinger (79) fährt, die an einer Decke (64) ausgeformt sind.

## Claims

1. Metering device (1) which can be activated by the user's suction airstream and is intended for the inhalation of a pulverulent substance (2), in particular of a medicinal kind, which is disposed in a storage chamber (15) and can be moved out of this chamber by means of a metering chamber (40) of a metering rod (33) into an emptying-standby position (B), in which position the metering chamber (40) is closed by a piston (54), which piston can be displaced in the direction of a mouthpiece (6), by means of the user's suction airstream, into an emptying-release position, **characterized in that** the piston (54), which is of disk-like configuration in the upper region, is provided with tongues (77) which extend from the underside of the disk, close the metering chamber (40) - or a plurality of metering chambers - in the emptying-standby position and release/open the metering chamber (40) when the piston is displaced by the user's suction airstream.

2. Metering device according to Claim 1, **characterized in that**, in its upper end position, the upper periphery of the piston (54) engages in front of an annular wall (80), which belongs to an annular chamber (63), which annular chamber (63) is provided with peripherally extending wings (65, 66) which leave intermediate spaces (67) between them, and **in that** an inclined deflecting wall (51) is provided above the ceiling (64) of the annular chamber (63).

3. Metering device according to Claim 2, **characterized in that** part of the wings (65) is of wider configuration in the circumferential direction, in order to form a deflecting-wall wing (73) for the suction airstream.

4. Metering device according to Claim 2, **characterized in that** a latching head (45) of the metering rod (33) is at least partially sunken into an upper depression of the piston (54), the piston being a disk piston.

5. Metering device according to Claim 3, **characterized by** the provision, on the lateral-wall side of an inner cylinder (53) which is moved by a closure cap (7), of an axially running channel (62) which extends from the emptying side of the metering chamber (40) and terminates in the annular chamber (63), the deflecting-wall wing (73) being provided in order to deflect the axial airstream direction into the circumferential plane.

6. Metering device according to Claim 1, **characterized in that**, along their lower free periphery, the tongues (77) are split in a lip-like manner in order to achieve a clamping action, and **in that** the tongues (77) have material-reinforced sealing surfaces (78).

7. Metering device according to Claim 1, **characterized in that** aligned with the metering chamber (40) - in the closed emptying-standby position - a channel (60) is directed toward one of the tongues (77), in particular in order to allow a visual check.

8. Metering device according to Claim 1, **characterized by** two air flows (a, b), of which the one opens the emptying-standby position (B) of the metering chamber (40) and the second leads directly into an annular chamber (63), which is located upstream of the mouthpiece (6) and where both airstreams meet.

9. Metering device according to Claim 8, **characterized in that** an air-inlet grille surface on an outer cylinder (4) is located on that side of the metering rod (33) which is located opposite to the emptying direction of the metering chamber (40), and **in that** a flow channel (60) directed toward the metering chamber (40) is disposed beneath the air-inlet grille surface, level with the position assumed by the metering chamber (40) in the emptying-standby position (B).

10. Metering device according to Claim 9, **characterized in that** an interior of an inner cylinder (53) is available entirely for the free distribution of the air sucked in through the air-inlet grille surface, and it is in flow connection with the annular chamber (63).

11. Metering device according to Claim 9, **characterized in that** the lateral wall of the outer cylinder (4) has at least one air-inlet opening (72), preferably two radially opposite air-inlet openings (72), and the air-inlet openings (72) open out in a tangentially directed manner into the annular chamber (63), a common flow direction being predetermined in the process.

12. Metering device according to Claim 1, **characterized by** an indicator (39), in the region of the storage-chamber wall, for indicating the actual filling level.

13. Metering device according to Claim 1, **characterized in that** the upward movement of a metering-chamber piston (16) is stopped.

14. Metering device according to Claim 1, **characterized in that** the metering rod (33) is latched in a disengageable manner in its upper position.

15. Metering device according to Claim 14, **characterized in that** a radial collar of the metering rod (33) moves behind latching fingers (79) which are formed on a ceiling (64).

## Revendications

1. Dispositif de dosage (1) pouvant être activé par le flux d'air d'aspiration d'un utilisateur pour l'inhalation d'une substance pulvérulente (2), en particulier de type médical, qui est disposée dans une chambre de stockage (15) et qui peut être amenée hors de celle-ci au moyen d'une chambre de dosage (40) d'une barre de dosage (33) dans une position prête à l'évacuation (B), dans laquelle position la chambre de dosage (40) est fermée par un piston (54), ce dernier pouvant être déplacé au moyen du flux d'air d'aspiration de l'utilisateur dans la direction d'un embouchoir (6) dans une position de libération d'évacuation, **caractérisé en ce que** le piston (54), configuré sous forme de plateau dans la région supérieure, est muni de langues (77) partant du côté inférieur du plateau, fermant la chambre de dosage (40) - ou plusieurs chambres de dosage - dans la position prête à l'évacuation, lesquelles langues libèrent/ouvrent la chambre de dosage (40) lors du déplacement du piston rendu possible par le flux d'air d'aspiration de l'utilisateur.

2. Dispositif de dosage selon la revendication 1, **caractérisé en ce que** le bord supérieur du piston (54), dans sa position d'extrémité supérieure, passe devant une paroi annulaire (80), qui appartient à une chambre annulaire (63), laquelle chambre annulaire (63) est munie d'ailes (65, 66) en déport du côté du bord, lesquelles laissent entre elles des espaces intermédiaires (67), et **en ce qu'**au-dessus du couvercle (64) de la chambre annulaire (63) est prévue une paroi d'impact (51) en position inclinée.

3. Dispositif de dosage selon la revendication 2, **caractérisé en ce qu'**une partie des ailes (65) est configurée plus large dans la direction périphérique pour former une aile de paroi d'impact de déflexion (73) pour le flux d'air d'aspiration.

4. Dispositif de dosage selon la revendication 2, **caractérisé en ce qu'**une tête d'encliquetage (45) de la barre de dosage (33) est enfoncée au moins en partie dans un renfoncement supérieur du piston (54), le piston étant un piston à plateau.

5. Dispositif de dosage selon la revendication 3, **caractérisé en ce que** du côté de la paroi d'enveloppe d'un cylindre intérieur (53) déplacé par un capuchon de fermeture (7) est prévu un canal s'étendant axialement (62), lequel part du côté d'évacuation de la chambre de dosage (40) et se termine dans la chambre annulaire (63), et l'aile de paroi d'impact de déflexion (73) étant prévue pour dévier la direction du flux d'air axial dans le plan périphérique.

6. Dispositif de dosage selon la revendication 1, **caractérisé en ce que** les langues (77) sont fendues en forme de lèvres au niveau de leur bord libre inférieur pour produire un effet de serrage, et **en ce que** les langues (77) possèdent des surfaces d'étanchéité (78) avec un épaississement de matériau.

7. Dispositif de dosage selon la revendication 1, **caractérisé en ce qu'**en alignement avec la chambre de dosage (40), - dans la position fermée prête à l'évacuation -, un canal (60) est orienté vers l'une des langues (77), en particulier pour permettre une possibilité de contrôle optique.

8. Dispositif de dosage selon la revendication 1, **caractérisé par** deux flux d'air (a, b), dont l'un ouvre la position prête à l'évacuation (B) de la chambre de dosage (40) et dont le deuxième conduit directement à une chambre annulaire (63) montée en amont de l'embouchoir (6), où les deux flux d'air se rejoignent.

9. Dispositif de dosage selon la revendication 8, **caractérisé en ce qu'**une surface en treillis d'entrée d'air est située au niveau d'un cylindre extérieur (4) sur le côté de la barre de dosage (33) qui est opposé à la direction d'évacuation de la chambre de dosage (40) et **en ce qu'**en dessous de la surface en treillis d'entrée d'air à la hauteur de la position adoptée par la chambre de dosage (40) dans la position prête à l'évacuation (B) est disposé un canal d'écoulement (60) orienté vers la chambre de dosage (40).

10. Dispositif de dosage selon la revendication 9, **caractérisé en ce qu'**un espace interne d'un cylindre intérieur (53) est complètement disponible pour la distribution libre de l'air aspiré par la surface en treillis d'entrée d'air et est en liaison d'écoulement avec la chambre annulaire (63).

11. Dispositif de dosage selon la revendication 9, **caractérisé en ce que** la paroi d'enveloppe du cylindre extérieur (4) possède au moins une, et de préférence deux, ouvertures d'entrée d'air (72) radialement opposées et les ouvertures d'entrée d'air (72) débouchent dans la chambre annulaire (63) en prédéfinissant une direction d'écoulement commune orientée tangentiellement.

12. Dispositif de dosage selon la revendication 1, **caractérisé par** un indicateur (39) associé au niveau de remplissage réel dans la région de la paroi de la chambre de stockage.

13. Dispositif de dosage selon la revendication 1, **caractérisé en ce que** le mouvement ascendant d'un piston de chambre de dosage (16) est bloqué.

14. Dispositif de dosage selon la revendication 1, **caractérisé en ce que** la barre de dosage (33) s'encliquète de manière déclenchable dans sa position relevée.

15. Dispositif de dosage selon la revendication 14, **caractérisé en ce qu'**un collet radial de la barre de dosage (33) passe derrière des doigts d'encliquetage (79), qui sont formés au niveau d'un couvercle (64).
